(19) 

Europäisches
Patentamt
European
Patent Office
Office européen
des brevets

(11) **EP 4 772 532 A1**

(12) **EUROPEAN PATENT APPLICATION**
published in accordance with Art. 153(4) EPC

(43) Date of publication:
08.07.2026 Bulletin 2026/28

(21) Application number: 24859995.3

(22) Date of filing: 30.08.2024

(51) International Patent Classification (IPC):
*C07K 14/775* (2006.01)   *A61K 38/41* (2006.01)
*A61K 41/00* (2020.01)   *A61P 27/02* (2006.01)
*A61P 43/00* (2006.01)   *C07K 14/795* (2006.01)
*C12N 15/12* (2006.01)

(52) Cooperative Patent Classification (CPC):
A61K 31/409; A61K 38/41; A61K 41/00;
A61K 45/00; A61K 47/24; A61K 47/42;
A61P 27/02; A61P 35/00; A61P 43/00;
C07K 14/435; C07K 14/775; C07K 14/795;
C12N 15/115

(86) International application number:
PCT/JP2024/031349

(87) International publication number:
WO 2025/047970 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.08.2023 JP 2023141459

(71) Applicant: Kyoto University
Kyoto-shi, Kyoto 606-8501 (JP)

(72) Inventors:
• MURAKAMI Tatsuya
Imizu-shi, Toyama 939-0398 (JP)
• SUDA Kenji
Kyoto-shi, Kyoto 606-8501 (JP)
• TSUJIKAWA Akitaka
Kyoto-shi, Kyoto 606-8501 (JP)

(74) Representative: Hoffmann Eitle
Patent- und Rechtsanwälte PartmbB
Arabellastraße 30
81925 München (DE)

Remarks:
The complete document including Reference
Table(s) and the Sequence Listing(s) can be
downloaded from the EPO website

(54) **PHOTOSENSITIVE-SUBSTANCE-CONTAINING LIPOPROTEIN, PHOTODYNAMIC THERAPY PREPARATION, DRUG DELIVERY METHOD, AND POSTERIOR OCULAR NEOVASCULAR OCCLUSION METHOD**

(57) It is an object of the present invention to provide a photodynamic therapy preparation with enhanced selectivity of the delivery site of a sensitive substance (drug). The present invention relates to a photosensitive substance-containing lipoprotein, comprising a modified high-density lipoprotein containing an apolipoprotein and a phospholipid, and a photosensitive substance, wherein the photosensitive substance-containing lipoprotein comprises one or more molecules of the photosensitive substance per particle of the modified high-density lipoprotein, and the apolipoprotein is formed by binding between a vascular endothelial cell-targeting peptide or aptamer and a cell affinity peptide.

[Figure 1]

VP@eLP1
NGR(−)

VP@eLP2
NGR(+)

Vis

**Description**

Technical Field

The present invention relates to a photosensitive substance-containing lipoprotein, a photodynamic therapy preparation, a drug delivery method, and a method for occluding new blood vessels in the posterior eye segment.

Background Art

Age-related macular degeneration (AMD) is a disease that progresses due to generation of new blood vessels from the choroid located outside the retina, resulting in a state of reduced vision. One treatment for the age-related macular degeneration is photodynamic therapy (PDT). In photodynamic therapy, a photosensitive substance such as verteporfin is delivered to the lesion site, and then, reactive oxygen generated from the photosensitive substance by photogeneration occludes new blood vessels, thereby stopping the progression of the disease.

For example, Patent Document 1 discloses a method for treating wet age-related macular degeneration (wAMD), in which a patient is treated by PDT using a photosensitizer. This document discloses administration of verteporfin as a photosensitizer. In addition, Patent Document 2 discloses a method for treating neovascular diseases of the eye, wherein the method comprises: administering at least one vascular vessel-occlusive substance containing at least one photosensitive substance that absorbs a light in the range of approximately 380 nm to approximately 720 nm to neovascularized tissues of the eye; and irradiating the eye with a light having a wavelength or waveband that matches the excitation wavelength or waveband of the photosensitive compound, thereby activating the photosensitive compound and occluding one or more vascular vessels in the neovascularized tissues of the eye for a sufficient occlusion period. Patent Document 2 discloses, as photosensitive compounds, porphyrin, purpurin, and verteporfin.

Non-Patent Document 1 discloses that photodynamic therapy is commonly used as a method for treating age-related macular degeneration, and that verteporfin (which is the component name of Visudyne®, 15 mg for intravenous injection, Novartis Pharma K.K.) is used as a photosensitive substance used for PDT. Visudyne is a liposomal formulation for treating age-related macular degeneration, the sales of which has been started from 2004. It is to be noted that the liposome that constitutes the liposomal formulation function as a drug carrier.

Besides, the present inventors have reported that a modified high-density lipoprotein comprising an apolipoprotein in which a vascular endothelial cell-targeting peptide or aptamer and a cell affinity peptide bind to each other, and a phospholipid, functions as a drug delivery carrier (Patent Document 3). However, this document does not specifically disclose that such a modified high-density lipoprotein is combined with a photosensitive substance, which is applied to photodynamic therapy.

Prior Art Documents

Patent Documents

Patent Document 1: JP Patent Publication No. 2017-537117 A
Patent Document 2: JP Patent Publication No. 2008-524224 A
Patent Document 3: JP Patent Publication No. 2021-138629 A

Non-Patent Documents

Non-Patent Document 1: Japanese Ophthalmological PDT Study Group "Guidelines for Photodynamic Therapy for Age-related Macular Degeneration," Journal of Japanese Ophthalmological Society, Vol. 108, No. 4

Summary of Invention

Objects to be Solved by the Invention

In photodynamic therapy, a photosensitive substance (drug) capable of generating reactive oxygen at the lesion site is encapsulated into a liposome and is delivered to the lesion site, followed by performing laser light irradiation limited to the lesion site. However, conventional age-related macular degeneration treatment agents have not had sufficiently high selectivity of the drug delivery site, and as a result, the drug has reached an area other than the lesion site in some cases. When the drug reaches an area other than the lesion site, if a laser light is incorrectly irradiated to such an area, normal tissues are also damaged and a reduction in vision, etc. may be induced, thereby causing problems. Furthermore, there is such a time constraint that a laser light must be irradiated 15 minutes after intravenous administration of the drug. For this reason, at present, in Japan, those practicing photodynamic therapy for age-related macular degeneration are recommended to obtain certification as certified doctors by attending a seminar co-sponsored by the Japan Retina and Vitreous Society and the Ophthalmology Photodynamic Therapy Study Group. A high level of expertise is required for the treatment of age-related macular degeneration.

If the selectivity of drug delivery site of age-related macular degeneration treatment agents could be enhanced, the delivery of the drug to areas other than the lesion site is suppressed. Thus, even if the laser light used in the treatment of age-related macular degeneration is irradiated to the entire eyeball, normal tissues are not damaged. Moreover, even if the laser light is not irradiated to the entire eyeball, the age-related macular degeneration treatment can be significantly simplified by irradiating the laser light, not to a pinpoint but to a wide area. Thus, in order to solve the problems associated with conventional age-related macular degeneration treatments, the present inventors have conducted studies for the purpose of enhancing the selectivity of the delivery site of a photosensitive substance (drug) in the age-related macular degeneration treatment, and enhancing the retentivity of the photosensitive substance (drug) at the diseased site. Furthermore, the present invention also aims to provide a photodynamic therapy preparation with enhanced selectivity of the delivery site of a photosensitive substance (drug) and enhanced retentivity of the photosensitive substance (drug) at the diseased site.

Means for Solving the Objects

Specific aspects of the present invention are described below.
[1] A photosensitive substance-containing lipoprotein, comprising a modified high-density lipoprotein containing an apolipoprotein and a phospholipid, and a photosensitive substance, wherein

the photosensitive substance-containing lipoprotein comprises one or more molecules of the photosensitive substance per particle of the modified high-density lipoprotein, and
the apolipoprotein is formed by binding between a vascular endothelial cell-targeting peptide or aptamer and a cell affinity peptide.

[2] The photosensitive substance-containing lipoprotein according to [1], wherein the photosensitive substance is one type or two or more types selected from the group consisting of a porphyrin compound, a chlorin compound, a bacteriochlorin compound, a phthalocyanine compound, and a cyanine dye.
[3] The photosensitive substance-containing lipoprotein according to [1] or [2], wherein the photosensitive substance is a porphyrin compound.
[4] The photosensitive substance-containing lipoprotein according to [3], wherein the porphyrin compound has a porphyrin ring and a hydrophilic group.
[5] The photosensitive substance-containing lipoprotein according to [3] or [4], wherein the porphyrin compound is verteporfin.
[6] The photosensitive substance-containing lipoprotein according to any one of [1] to [5], which has a volume average particle diameter of 140 nm or less.
[7] The photosensitive substance-containing lipoprotein according to any one of [1] to [6], wherein the vascular endothelial cell-targeting peptide or aptamer is a neovascular endothelial cell-targeting peptide or aptamer.
[8] The photosensitive substance-containing lipoprotein according to [7], wherein the neovascular endothelial cell-targeting peptide is one type or two or more types selected from the group consisting of an NGR-containing peptide, an RGD-containing peptide, and a CPRECES-containing peptide.
[9] The photosensitive substance-containing lipoprotein according to any one of [1] to [8], wherein the cell affinity peptide is one type or two or more types selected from the group consisting of a TAT peptide, penetratin (PEN), polyarginine (R8), LL-37, transportan, Pep-1, and MTS.
[10] A photodynamic therapy preparation, comprising the photosensitive substance-containing lipoprotein according to any one of any one of [1] to [9].
[11] The photodynamic therapy preparation according to [10], which is used in the treatment of age-related macular degeneration.
[12] The photodynamic therapy preparation according to [10] or [11], wherein the photodynamic therapy comprises administering the photosensitive substance-containing lipoprotein to a subject who is affected with age-related macular degeneration, and applying light irradiation to a new blood vessel generation site in the posterior eye segment.

[13] A method for occluding new blood vessels in the posterior eye segment, wherein the method comprises administering the photosensitive substance-containing lipoprotein according to any one of [1] to [9] to a subject who is affected with age-related macular degeneration, and applying light irradiation to a new blood vessel generation site in the posterior eye segment.

[14] The photodynamic therapy preparation according to [10], which is used in the treatment of malignant tumor.

[15] The photodynamic therapy preparation according to [14], wherein the photodynamic therapy comprises administering the photosensitive substance-containing lipoprotein to a subject having malignant tumor, and applying light irradiation to a new blood vessel generation site in the malignant tumor part.

[16] A method for occluding new blood vessels in a malignant tumor part, wherein the method comprises administering the photosensitive substance-containing lipoprotein according to any one of [1] to [9] to a subject having malignant tumor, and applying light irradiation to a new blood vessel generation site in the malignant tumor part.

[A] The photosensitive substance-containing lipoprotein according to any one of [1] to [9], which is for use in photodynamic therapy.

[B] Use of the photosensitive substance-containing lipoprotein according to any one of [1] to [9] for production of a photodynamic therapy preparation.

[C] The photosensitive substance-containing lipoprotein according to any one of [1] to [9], which is for use in the treatment of age-related macular degeneration.

[D] Use of the photosensitive substance-containing lipoprotein according to any one of [1] to [9] for production of a preparation for the treatment of age-related macular degeneration.

[E] The photosensitive substance-containing lipoprotein according to any one of [1] to [9], which is for use in the treatment of malignant tumor.

[F] Use of the photosensitive substance-containing lipoprotein according to any one of [1] to [9] for production of a preparation for the treatment of malignant tumor.

[G] The photosensitive substance-containing lipoprotein according to any one of [1] to [9], which is for use in a method for occluding new blood vessels in the posterior eye segment.

[F] The photosensitive substance-containing lipoprotein according to any one of [1] to [9], which is for use in a method for occluding new blood vessels in a malignant tumor part.

Advantageous Effects of Invention

According to the present invention, the selectivity of the delivery site of a photosensitive substance (drug) can be enhanced in the treatment of age-related macular degeneration. In addition, according to the present invention, a photodynamic therapy preparation with enhanced selectivity of the delivery site of a photosensitive substance (drug) can be provided.

Brief Description of Drawings

[Figure 1] Figure 1 is a schematic view illustrating the structure of the photosensitive substance-containing lipoprotein of the present embodiment (central view). The left view is a schematic view illustrating the structure of a conventional modified high-density lipoprotein, and the right view is a schematic view illustrating the structure of the existing formulation (liposomal formulation).

[Figure 2] Figure 2 shows the VP absorption spectrum of each nanoparticle that loaded verteporfin (VP).

[Figure 3] Figure 3 is a graph showing the ability to generate singlet oxygen ($^1O_2$).

[Figure 4] Figure 4 shows images in which the amount of each nanoparticle incorporated into HUVEC cells was observed, and a graph showing the weight of VP in the cells.

[Figure 5] Figure 5 is a graph showing the cytotoxicity (phototherapeutic effect) of HUVEC cells incorporated with each nanoparticle, when a light was irradiated to the cells.

[Figure 6] Figure 6 is a graph showing the blood retention time of each nanoparticle after intravenous administration of each nanoparticle into mice.

[Figure 7] Figure 7 shows images in which accumulation of each nanoparticle in new blood vessels in the choroid of the posterior eye segment (choroidal neovascularization; CNV) was observed after intravenous administration of each nanoparticle into mice.

[Figure 8] Figure 8 is a graph showing the CNV retention time in Figure 7.

[Figure 9] Figure 9 shows observation images showing the CNV area after intravenous administration of each nanoparticle into mice and laser light irradiation, and a graph showing the area of the CNV area.

[Figure 10] Figure 10 shows observation images of the entire eyeball (left) and the retina near the optic disc (right) after intravenous injection of each nanoparticle to mice and laser light irradiation.

[Figure 11] Figure 11 is a graph showing the fluorescence intensity of NBD in HUVEC cells incorporated with each nanoparticle in the presence of a CD13 inhibitor.

[Figure 12] Figure 12 shows a graph showing the fluorescence intensity of VP in HUVEC cells incorporated with each nanoparticle in the presence of a CD13 inhibitor.

[Figure 13] Figure 13 shows a graph showing the analysis of the mRNA expression levels of various types of transporters in HUVEC cells incorporated with each nanoparticle.

[Figure 14] Figure 14 shows a graph showing the tumor volume increase percentage after photodynamic therapy has been applied to cancer mouse models.

Embodiments of Carrying out the Invention

The present invention will be described in detail below. The explanation given below may be described based on some typical embodiments or specific examples. However, the present invention should not be limited to such embodiments. It is to be noted that, in the present description, the numerical range expressed using the preposition "to" means a range that includes the numbers before and after "to" as the lower limit value and the upper limit value.

(Photosensitive substance-containing lipoprotein)

The present embodiment relates to a photosensitive substance-containing lipoprotein, comprising a modified high-density lipoprotein containing an apolipoprotein and a phospholipid, and a photosensitive substance, wherein the photosensitive substance-containing lipoprotein comprises one or more molecules of the photosensitive substance per particle of the modified high-density lipoprotein, and the apolipoprotein is formed by binding between a vascular endothelial cell-targeting peptide or aptamer and a cell affinity peptide.

The high-density lipoprotein (HDL) that constitutes the photosensitive substance-containing lipoprotein comprises an apolipoprotein A-I (apoA-I) and a phospholipid. The high-density lipoprotein (HDL) may be either a natural plasma-derived high-density lipoprotein, or a reconstituted (i.e., artificial) high-density lipoprotein (rHDL) produced by a chemical synthesis method or a genetic engineering method using an apolipoprotein or a genetically modified apolipoprotein thereof and a phospholipid. The high-density lipoprotein (HDL) used in the present embodiment comprises an apolipoprotein and a phospholipid as main components. When a high-density lipoprotein (HDL) is artificially prepared, several ten times to several hundred times the molar amount of the phospholipid can be used per mole of the apolipoprotein.

The density of the high-density lipoprotein (HDL) is preferably within the range from approximately 1.063 to 1.210 g/mL when it is naturally occurring HDL (see Antonio M. Gotto, Jr. et al., Methods Enzymol. 1986; 128: 3-41). On the other hand, in the case of rHDL, the size thereof conforms to naturally occurring HDL, but the density thereof can be adjusted to a desired density upon production, and the density is preferably 1.063 g/mL or more.

The apolipoprotein includes an apolipoprotein generally known to be contained in a natural lipoprotein and a genetically modified apolipoprotein thereof, but examples of the apolipoprotein are not limited thereto. Among others, the apolipoprotein preferably includes an apolipoprotein known to be contained in a high-density lipoprotein (HDL) and a genetically modified apolipoprotein thereof. Examples of the apolipoprotein may include proteins belonging to the group consisting of apolipoproteins A to F, H, J, L-I, M and O. The apolipoprotein is preferably one type or two or more types selected from the group consisting of an apolipoprotein A-I (apoA-I), an apolipoprotein A-II (apoA-II), an apolipoprotein C (apoC), an apolipoprotein E (apoE), and their genetically modified apolipoproteins, and is more preferably one type or two types selected from an apolipoprotein A-I (apoA-I) and genetically modified apoA-I thereof. The genetically modified apolipoprotein refers to a variant or an analogue, etc. (i.e., a functional equivalent) having the same function as that of the apolipoprotein (e.g., a lipid-binding function). Examples of the genetically modified apolipoproteins may include partial fragments of apolipoproteins and apolipoproteins combining these fragments. An example of the genetically modified apolipoprotein of apolipoprotein A-I is apoA-I lacking 44 amino acids at the N-terminus.

In the present embodiment, the apolipoprotein is formed by binding between a vascular endothelial cell-targeting peptide or aptamer and a cell affinity peptide. It is to be noted that, in the present description, the apolipoprotein formed by binding between a vascular endothelial cell-targeting peptide or aptamer and a cell affinity peptide is also referred to as a modified apolipoprotein or an apolipoprotein variant.

The vascular endothelial cell-targeting peptide or aptamer (hereinafter also abbreviated as "VTP") means a peptide or aptamer that can bind to vascular endothelial cells and can target blood vessels. VTP is preferably a "neovascular endothelial cell-targeting peptide or aptamer" that can bind to neovascular endothelial cells and can target vascular endothelial cells, and is particularly preferably a neovascular endothelial cell-targeting peptide. For example, it is known that $\alpha_v\beta_3$ integrin, aminopeptidase A, aminopeptidase N (APN; also called CD13), or a vascular endothelial growth factor (VEGF) receptor, etc. is expressed in new blood vessels (J. Lahdenranta et al. (2007) The FASEB Journal; 21: 3272-3278). Therefore, the neovascular endothelial cell-targeting peptide and aptamer include antibodies, their fragments, and aptamers, etc. that specifically bind to these molecules.

Examples of the neovascular endothelial cell-targeting peptide may include one type or two or more types selected from the group consisting of an "asparagine-glycine-arginine (NGR)-containing peptide," an RGD-containing peptides such as ACDCRGDCFC (SEQ ID NO: 2), and a CPRECES-containing peptide such as CPKVCPRECESNC (SEQ ID NO: 3). These peptides are molecules discovered as tumor new blood vessel targeting peptides that bind to the CD13 receptor, $\alpha_v\beta_3$ integrin, or aminopeptidase A, respectively, on tumor vascular endothelial cells, and have been reported to be bindable also to new blood vessels in the retina (J. Lahdenranta et al. (2007) supra). These peptides may have any amino acid added before or after them, as long as it does not impair their ability to bind to new blood vessels. For example, the NGR-containing peptide may be either NGR alone, or $X_m NGRY_n$ with other amino acids attached to one or both of before and after NGR. Here, X is any amino acid, m is an integer from 1 to 10, and when m is 2 or greater, X is a combination of the same or different amino acids. Y is any amino acid, n is an integer from 1 to 10, and when n is 2 or greater, Y is a combination of the same or different amino acids. An example of a peptide represented by $X_m NGRY_n$ may be CNGRCGG (SEQ ID NO: 4). The presence of cysteines (C) before and after NGR allows for formation of an S-S bond by oxidation, thereby cyclizing the NGR moiety and enhancing receptor binding activity.

The NGR-containing peptide is also expected to have the function of enhancing drug retentivity in CD13-positive cells. Thereby, the disease site retentivity of the photosensitive substance-containing lipoprotein can be enhanced, and time limitation in photodynamic therapy can be alleviated.

The antibody specifically binding to a molecules selected from a VEGF receptor may be, for example, bevacizumab (anti-VEGF humanized monoclonal antibody). In addition, the antibody can also include a VHH (variable domain of heavy chain of heavy chain antibody) antibody, and examples of the antibody fragments may include, for example, Fab, F(ab')2, Fab', diabody, and single-chain antibodies (e.g., scFv, dsFv).

The vascular endothelial cell-targeting aptamer is preferably, for example, a DNA aptamer or an RNA aptamer. For example, the aptamer binding to a VEGF receptor may include the $Apt_{80mer}$, $Apt_{M80mer}$, and $APT_{Divalent}$ described in NUCLEIC ACID THERAPEUTICS, Volume 25, Number 5, pages 227-234, 2015 (see Table 1 below). The 5 in the nucleotide sequence of the $Apt_{80mer}$ represents a benzyl-containing nucleotide (BndU). The $Apt_{M80mer}$ is shown in SEQ ID NO: 5.

[Table 1]

| Aptamer | Description | Sequence |
|---|---|---|
| $Apt_{80mer}$ | 80mer monomer | 5' GAT GTG AGT GTG TGA CGA GC5 ACG ACG 5C5 GG5 G5A A55 5A5 AAA GAC AC5 G5G 5A5 A5C AAC AAC AGA ACA AGG AAA GG 3' |
| $Apt_{M80mer}$ | Modified 80mer monomer | 5' GAT GTG AGT GTG TGA CGA GCT ACG ACG TCT GGT GTA ATT TAT AAA GAC ACT GTG TAT ATC AAC AAC AGA ACA AGG AAA GG 3' |
| $Apt_{Divalent}$ | 5'-3'/spacer/5'-3' | 5' GAT GTG AGT GTG TGA CGA GCT ACG ACG TCT GGT GTA ATT TAT AAA GAC ACT GTG TAT ATC AAC AAC AGA ACA AGG AAA GG 3'/ (PEG)$_6$ spacer /5' GAT GTG AGT GTG TGA CGA GCT ACG ACG TCT GGT GTA ATT TAT AAA GAC ACT GTG TAT ATC AAC AAC AGA ACA AGG AAA GG 3' |

The cell affinity peptide (hereinafter abbreviated as "CP" at times) is a peptide that exhibits cell affinity by which it can bind to the cell membrane and then can migrate into the cell. In the present description, the cell affinity includes, but is not limited to, cell membrane permeability, cell adhesion, vascular endothelial cell accumulation, endosomal escape, nuclear accumulation, and mitochondrial accumulation.

The CP includes a natural peptide derived from a natural protein and an artificially produced synthetic peptide (e.g., a chimeric peptide). The CPs are classified into a basic cell affinity peptide, an amphipathic cell affinity peptide, and a hydrophobic cell affinity peptides, according to the chemical properties of their amino acid sequences. Examples of the basic cell affinity peptide may include, but are not limited to, the TAT peptide derived from the TAT protein (Trans-activator of transcription protein) of the HIV-1 virus, penetratin (PEN) derived from Drosophila, polyarginine (R8), and LL-37 derived from an antibacterial peptide. Examples of the amphipathic cell affinity peptide may include, but are not limited to, the chimeric peptides transportan and Pep-1, and modified or shuffled bodies of PEN sequences. Examples of the hydrophobic cell affinity peptide may include, but are not limited to, Pep-1, a synthetic peptide obtained by a phage display method, and MTS, a peptide derived from the signal peptide of a membrane protein. Among these, the CPs used in the present embodiment are preferably one type or two or more types selected from the group consisting of a TAT peptide, penetratin (PEN), polyarginine (R8), LL-37, transportan, Pep-1, and MTS. In particular, the CPs used in the present embodiment are preferably basic cell affinity peptides, more preferably one type or two or more types selected from the group consisting of an TAT peptide, penetratin (PEN), polyarginine (R8), and LL-37, and particularly preferably penetratin (PEN).

VTP and CP bind to an apolipoprotein to form a VTP- and CP-bound apolipoprotein. In the present description, such a VTP- and CP-bound apolipoprotein is also referred to as "vc-apo."

In a modified apolipoprotein (vc-apo), at least one VTP and at least one CP bind to an apolipoprotein. When one VTP and one CP bind to the apolipoprotein, it is preferable that the VTP binds to the N-terminus of the apolipoprotein, and that the CP binds to the C-terminus of the apolipoprotein. Otherwise, it is also possible that the VTP binds to the C-terminus of the apolipoprotein, and that the CP binds to the N-terminus of the apolipoprotein. Alternatively, both the VTP and the CP can bind to either the N-terminus or the C-terminus of the apolipoprotein. When two or more VTPs bind to the apolipoprotein, the two or more VTPs can bind thereto consecutively. When two or more CPs bind to the apolipoprotein, the two or more CPs can bind thereto consecutively. Otherwise, the VTP and the CP can each bind to both the N-terminus and the C-terminus of the apolipoprotein. Binding two or more VTPs and/or CPs to a single apolipoprotein can enhance vascular targeting ability and/or cell affinity in some cases. The modified high-density lipoprotein may comprise two or more molecules of apolipoproteins, and each apolipoprotein can comprise two or more molecules of VTPs and CPs, respectively.

Herein, as a method of binding the VTP and the CP to the apolipoprotein, a chemical synthesis method (e.g., a coupling method) or a biological method (e.g., a genetic engineering method) can be adopted. When the VTP and the CP are artificially bound to an apolipoprotein by producing a transforming (fusion) gene according to a genetic engineering method, the VTP and the CP bind to the apolipoprotein via "fusion." The functional groups fused and the positions thereof on the apolipoprotein can be modified, as appropriate, depending on the VTP and the CP to be fused.

The content of the modified apolipoprotein in the modified high-density lipoprotein is preferably 0.01 $\mu$mol/L or more, and more preferably 0.05 $\mu$mol/L or more. On the other hand, the content of the modified apolipoprotein in the modified high-density lipoprotein is preferably 0.5 $\mu$mol/L or less, and more preferably 0.1 $\mu$mol/L or less.

Moreover, the modified apolipoprotein preferably comprises the VTP and the CP, which are fused to the apolipoprotein at an equal molar ratio. However, two or more molecules of the VTPs and the CPs are also allowed to bind to one molecule of the apolipoprotein, respectively, and in such a case, the modified apolipoprotein comprises the VTP and/or the CP, which are fused to the apolipoprotein at a molar ratio of two or more times.

The modified high-density lipoprotein comprises a phospholipid. The phospholipid means a lipid having one or multiple phosphoric acid ester sites. The phospholipid in the present description includes a phospholipid generally known to be comprised in a natural lipoprotein. A phospholipid known to be comprised in a high-density lipoprotein (HDL) is preferable, but the present phospholipid is not limited thereto. The phospholipid may be, for example, a sphingolipid having a sphingosine as a skeleton, and the number of carbon atoms of the acyl group is preferably 12 to 18, and more preferably 14 to 18. Examples of the phospholipid may include phosphatidylglycerol, phosphatidylserine, phosphatidylcholine, and phosphatidylethanolamine, with phosphatidylcholine being preferred. Specific examples may include, but are not limited to, dimyristoyl phosphatidylcholine (DMPC), dipalmitoyl phosphatidylcholine (DPPC), distearoyl phosphatidylcholine (DSPC), egg phosphatidylcholine (PC), 1-palmitoyl-2-oleoyl phosphatidylcholine (POPC), and hydrogenated soybean phosphatidylcholine (HSPC). It is to be noted that the hydrogenated soybean phosphatidylcholine (HSPC) may contain approximately 85% distearoyl phosphatidylcholine (DSPC). The phospholipid may be one to which saturated fatty acid binds. The phospholipid may be one type or two or more types selected from the group consisting of dimyristoyl phosphatidylcholine (DMPC), dipalmitoyl phosphatidylcholine (DPPC), distearoyl phosphatidylcholine (DSPC), and hydrogenated soybean phosphatidylcholine (HSPC), or may be one type or two or more types selected from distearoyl phosphatidylcholine (DSPC) and hydrogenated soybean phosphatidylcholine (HSPC). For example, by using a phospholipid to which saturated fatty acid binds, it is considered that reactive oxygen generation ability at a lesion site can be more effectively enhanced. Besides, the phospholipid can be used alone or in combination of two or more types.

In the phospholipid, for example, it is easy to adjust the hardness of the lipid membrane, and a stabilized complex can be formed using the phospholipid. Moreover, the phospholipid plays a role in contributing to encapsulation of a compound such as a drug. Furthermore, cholesterol can also be added to the phospholipid before use. By allowing the phospholipid to contain cholesterol, the hardness of the lipid membrane formed by the phospholipid can be adjusted. By adding cholesterol to the phospholipid, the phospholipid can interact with the active ingredient of a drug, etc., and adjust the encapsulation efficiency in the lipid membrane can also be adjusted. The amount of cholesterol added to the phospholipid is, for example, preferably in the range of 0.01% to 30% by mass.

The content of the phospholipid in the modified high-density lipoprotein is preferably 0.5 $\mu$mol/L or more, more preferably 1.0 $\mu$mol/L or more, even more preferably 1.5 $\mu$mol/L or more, further preferably 2.0 $\mu$mol/L or more, and particularly preferably 2.5 $\mu$mol/L or more. On the other hand, the content of the phospholipid in the modified high-density lipoprotein is preferably 250 $\mu$mol/L or less, more preferably 200 $\mu$mol/L or less, even more preferably 150 $\mu$mol/L or less,

further preferably 100 $\mu$mol/L or less, still further preferably 50 $\mu$mol/L or less, and particularly preferably 20 $\mu$mol/L or less.

Furthermore, the content of the phospholipid in the modified high-density lipoprotein is preferably 0.1 mg/mL or more, more preferably 1 mg/mL or more, and even more preferably 2 mg/mL or more, per 1 mg/mL of the modified apolipoprotein. The content of the phospholipid in the modified high-density lipoprotein is preferably 100 mg/mL or less, more preferably 50 mg/mL or less, and even more preferably 20 mg/mL or less, per 1 mg/mL of modified apolipoprotein.

The photosensitive substance-containing lipoprotein of the present embodiment comprises one or more molecules of the photosensitive substance, per particle of the modified high-density lipoprotein. The photosensitive substance generates reactive oxygen and heat upon light irradiation. In the treatment of age-related macular degeneration, the reactive oxygen and heat generated from the photosensitive substance can occlude new blood vessels generated from the choroid located outside the retina, thereby stopping progression of the disease. In particular, the photosensitive substance used in the present embodiment preferably generates reactive oxygen through light.

The photosensitive substance preferably absorbs a light with a wavelength in the range of 380 to 850 nm. For example, the absorption wavelength of verteporfin is approximately 690 nm, the absorption wavelength of indocyanine green (ICG) is approximately 800 nm, and the absorption wavelength of porfimer sodium is approximately 630 nm.

Examples of the photosensitive substance may include a porphyrin compound, a chlorin compound, a bacteriochlorin compound, a phthalocyanine compound, and a cyanine dye. More specifically, examples of the photosensitive substance may include a hematoporphyrin derivative, porfimer sodium, talaporfin sodium, protoporphyrin IX (PpIX), pheophorbide (Pheophorbide a), indocyanine green, aluminum phthalocyanine tetrasulfate (AlPcS$_4$), tin etiopurpurin (SnET$_2$), zinc(II) octadecylphthalocyanine (ZnOPPc), purpurinimide, azachlorin, zinc etiopurpurin (ZnET$_2$), phthalocyanine (Pc), cadmium texaphyrin (CdTX), texaphyrin, zinc tetraphyptaloporphyrin (ZnTNP), verdain, benzoporphyrin derivative monoacid ring A (BPDMA), purpurin, zinc tetrasulfonated phthalocyanine (ZnTSPc), gallium phthalocyanine (Ga-Pc), indium phthalocyanine (In-Pc), a benzoporphyrin derivative (BPD), calcium sulfonated phthalocyanine (Ca-SPc), a zinc phthalocyanine derivative (ZnPc), aluminum sulfonated phthalocyanine (AlSPc), a benzoporphyrin derivative, N-aspartyl chlorin e6 (Npe6), methylene blue, verteporfin, rhodamine, temoporphyrin, porphycene, hypersine, and IR-700. These photosensitive substances may be contained in a part of a complex, and for example, a complex may be formed by binding the photosensitive substance to an antibody or the like. Among others, the photosensitive substance is preferably a porphyrin compound or a cyanine dye, and is more preferably one type or two or more types selected from the group consisting of porfimer sodium, indocyanine green, verteporfin, N-aspartyl chlorin e6 (Npe6), and IR-700, and is particularly preferably verteporfin.

The porphyrin compound preferably has a porphyrin ring exhibiting hydrophobicity and a hydrophilic group. When the photosensitive substance has a porphyrin ring exhibiting hydrophobicity and a hydrophilic group, the photosensitive substance is stably encapsulated into the phospholipid. Examples of the hydrophilic group that can be possessed by the porphyrin compound may include a carboxylic acid group, a sulfonic acid group, a phosphoric acid group, a phosphonic acid groups, and a hydroxy group.

The content of the photosensitive substance in the photosensitive substance-containing lipoprotein is preferably 0.001 mol or more, more preferably 0.005 mol or more, and even more preferably 0.01 mol or more, per mol of the phospholipid. The upper limit value of the content of the photosensitive substance in the photosensitive substance-containing lipoprotein is not particularly limited, and it is preferably 5 mol or less, more preferably 3 mol or less, and even more preferably 1 mol or less, per mol of the phospholipid.

The content of the photosensitive substance in the photosensitive substance-containing lipoprotein is preferably 0.1 mol or more, more preferably 0.5 mol or more, and even more preferably 1 mol or more, per mol of modified apolipoprotein. The upper limit value of the content of the photosensitive substance in the photosensitive substance-containing lipoprotein is not particularly limited, and it is preferably 500 mol or less, more preferably 300 mol or less, and even more preferably 100 mol or less, per mol of modified apolipoprotein.

With regard to quantification of the photosensitive substance contained in the photosensitive substance-containing lipoprotein, the amount of the photosensitive substance contained in the photosensitive substance-containing lipoprotein can be calculated from the integrated fluorescence intensity in the fluorescence spectrum of the photosensitive substance.

In a preferred embodiment, for example, the photosensitive substance-containing lipoprotein comprises approximately 5 mg/mL of the phospholipid and approximately 0.05 mg (50 $\mu$g)/mL of the photosensitive substance, per 1 mg/mL of the modified apolipoprotein. In addition, in a preferred embodiment, the photosensitive substance-containing lipoprotein comprises 50 to 200 mol of the phospholipid and 1 to 50 mol of the photosensitive, substance per mol of the modified apolipoprotein.

The photosensitive substance-containing lipoprotein of the present embodiment may comprise, in addition to the photosensitive substance, other drugs, fluorescently labeled substances, bioactive substances, etc. In this case, the content of the other drugs, fluorescently labeled substances, or bioactive substances may be, for example, 0.01 to 25 $\mu$mol/L or 0.05 to 2 $\mu$mol/L.

In the modified high-density lipoprotein (hereinafter abbreviated as "vcHDL" at times), which comprises the modified apolipoprotein and the phospholipid, the apolipoprotein contains VTP in addition to CP, which allows vcHDL to function as a drug delivery carrier to the posterior eye segment, and to target new blood vessels. This allows the modified high-density lipoprotein to have excellent delivery ability to new blood vessels in the posterior eye segment, which is the affected area of age-related macular degeneration, which seems to be caused by abnormal neovascularization. Thus, by loading a photosensitive substance, a drug for treating age-related macular degeneration, onto vcHDL, the drug (photosensitive substance) can be selectively delivered to the affected area of age-related macular degeneration. Since the photosensitive substance-containing lipoprotein of the present embodiment is the above-described modified high-density lipoprotein (vcHDL) on which a photosensitive substance is loaded, the drug can be selectively delivered to the affected area of age-related macular degeneration. Thereby, the delivery efficiency of the drug (photosensitive substance) can be enhanced. It is to be noted that the delivery efficiency of the drug (photosensitive substance) in the present description refers to the proportion of the administered drug (photosensitive substance) that reaches new blood vessels in the posterior eye segment, the area affected by age-related macular degeneration. By enhancing the delivery efficiency of the drug (photosensitive substance), it becomes possible to increase the amount of the drug (photosensitive substance) that reaches new blood vessels in the posterior eye segment, and as a result, the intensity of light irradiation can be reduced, and also, the safety of the treatment can be further enhanced. Furthermore, by selectively delivering the drug to the affected area, generation of phototoxicity (side effects) can be suppressed, even in a case where a light is irradiated to areas other than the lesion site. Since this allows for a wide range of light irradiation, it becomes possible to significantly simplify the treatment of age-related macular degeneration. Furthermore, by increasing the disease site retentivity of the drug, the time limit (15 minutes) between intravenous administration and laser light irradiation cannot only be relaxed, but the clearance of the photosensitive compound from normal tissue can also be promoted, thereby reducing side effects.

The volume average particle diameter of the photosensitive substance-containing lipoprotein of the present embodiment is preferably 140 nm or less, more preferably 100 nm or less, even more preferably 75 nm or less, further preferably 50 nm or less, and particularly preferably 30 nm or less. It is to be noted that the lower limit value of the volume average particle diameter of the photosensitive substance-containing lipoprotein is not particularly limited, and for example, it is preferably 5 nm or greater. The smaller the size of the photosensitive substance-containing lipoprotein, the greater its potential for delivery to the posterior eye segment that can be expected. Therefore, by setting the volume average particle diameter of the photosensitive substance-containing lipoprotein within the above-described range, the delivery efficiency of the drug (photosensitive substance) can be more effectively enhanced.

Besides, since the volume average particle diameter of the photosensitive substance-containing lipoprotein is small, as mentioned above, and it is on the nanometer order, the photosensitive substance-containing lipoprotein is also referred to as a "nanoparticle" at times in the present description.

The volume average particle diameter and surface charge of the photosensitive substance-containing lipoprotein can be measured using a Nanotrac UPA-UT151 particle size analyzer (MicrotracBEL Corp., Tokyo, Japan) and a ZETASIZER NANO Z (Malvern, Worcestershire, U.K.), respectively.

The reactive oxygen (singlet oxygen) generation ability of the photosensitive substance-containing lipoprotein of the present embodiment, which is calculated using the expression shown below, is preferably 400% or higher, more preferably 450% or higher, and even more preferably 500% or higher. It is to be noted that the upper limit value of the reactive oxygen (singlet oxygen) generation ability of the photosensitive substance-containing lipoprotein is not particularly limited, and it is, for example, preferably 3000% or less.

[Expression 1]

$$(F - F_0) / F_0 \times 100 \ (\%)$$

The reactive oxygen (singlet oxygen) generation ability is detected using Singlet Oxygen Sensor Green Reagent (SOSG; Invitrogen Thermo Fisher Scientific Inc., USA), which selectively reacts with $^1O_2$ and emits fluorescence. Specifically, a solution containing a photosensitive substance-containing lipoprotein and SOSG is prepared, and this solution is irradiated with a light (10 mW, 5 min) using a 689 nm xenon lamp (Asahi Spectra, Tokyo, Japan). The fluorescence spectra of the solution before and after the light irradiation are measured using a plate reader (TECAN) (Ex. 480 nm / Em. 500 to 560 nm), and the integrated fluorescence intensity is then calculated. The integrated fluorescence intensity before the light irradiation is set as $F_0$, and the integrated fluorescence intensity after the light irradiation is set as F. The $^1O_2$ generation ability (%) is calculated using the above formula.

In the present embodiment, for example, the reactive oxygen (singlet oxygen) generation ability can be more effectively enhanced, for example, by using a phospholipid formed by binding saturated fatty acid thereof, and the reactive oxygen generation ability can be more effectively enhanced at the lesion site. If the reactive oxygen (singlet oxygen) generation ability can be increased, it will be also possible to reduce the loaded amount and the administered dose of the drug.

(Method for producing photosensitive substance-containing lipoprotein)

The present embodiment relates to a method for producing a photosensitive substance-containing lipoprotein, comprising the following steps i) to v):

i) fusing VTP and CP to an apolipoprotein by a genetic engineering method to obtain a fusion protein (modified apolipoprotein);
ii) producing crude vcHDL by mixing the resulting fusion protein with a liposome containing a phospholipid (spontaneous interaction method); or, alternatively, producing crude vcHDL by mixing the resulting fusion protein with cholate micelles without liposome formation (cholate-dialysis method);
iii) purifying the crude vcHDL by removing unreacted phospholipid and apolipoprotein;
iv) mixing the photosensitive substance with vcHDL to produce a photosensitive substance-containing lipoprotein containing one or more molecules of the photosensitive substance per vcHDL particle; and
v) purifying the photosensitive substance-containing lipoprotein by removing unreacted photosensitive substance.

In the step i), a genetic engineering method can be used. Examples of the genetic engineering method may include DNA editing using methylase or DNA polymerase I/DNA ligase, and a polymerase chain reaction (PCR) method. Specifically, a penetratin (PEN) peptide gene with a Pst I recognition sequence at the 5'-terminus and an Xba I recognition sequence at the 3-terminus is inserted into the Pst I and Xba I restriction enzyme recognition sites of the *E. coli* expression vector pCOLD I, using DNA ligase. Next, an N-terminal 44-amino acid deleted apoA-I gene with a Kpn I restriction enzyme recognition sequence at the 5'-terminus and a Pst I restriction enzyme recognition sequence at the 3'-terminus is produced by the PCR method, and the thus produced gene is then inserted between the Kpn I and Pst I recognition sites of the above-described pCOLD I, using DNA ligase. Through these operations, a peptide gene, in which a PEN peptide gene is fused with the C-terminus of the N-terminal 44-amino acid deleted apoA-I and an NGR peptide gene is fused with the N-terminus thereof, can be produced. When a VTP- and CP-bound apolipoprotein (modified apolipoprotein) is produced, a fusion protein gene is produced by adding an equimolar ratio of the VTP gene and the CP gene to the apolipoprotein gene before the fusion.

In the step ii), a modified high-density lipoprotein (vcHDL) is prepared by adding several times to several thousand times of moles, several ten times to several hundred times of moles, for example, approximately 5 times to approximately 2000 times of moles, approximately 20 times to approximately 500 times of moles, or approximately 30 times to approximately 200 times of moles of phospholipids per mole of the fusion protein.

The particle size of the modified high-density lipoprotein (vcHDL) produced above can be adjusted by adjusting the above-described molar ratio between the fusion protein and the phospholipid and further, by obtaining a desired density fraction after density gradient ultracentrifugation. However, the method of adjusting the particle size of vcHDL is not limited to these methods.

In the step iii), by using gel filtration or an ultracentrifugation method, unreacted phospholipids and apolipoproteins can be removed, crude vcHDL can be purified to obtain vcHDL.

In the step iv), the photosensitive substance is mixed with vcHDL. The amount of the photosensitive substance added is preferably 0.001 mol or more, more preferably 0.01 mol or more, and even more preferably 0.03 mol, per mol of the phospholipid. The amount of the photosensitive substance added is preferably 100 mol or less, more preferably 50 mol or less, and even more preferably 25 mol or less, per mol of phospholipid.

The mixing temperature in the step iv) is preferably 30°C to 70°C, and more preferably 40°C to 60°C. The mixing time is preferably 1 to 100 minutes, and more preferably 1 to 60 minutes. In the present embodiment, by using a compound having a porphyrin ring and a hydrophilic group as a photosensitive substance, the affinity between vcHDL and the photosensitive substance can be enhanced, and as a result, the mixing temperature in the step iv) can be lowered, and the mixing time can be shortened. Thereby, the production efficiency of the photosensitive substance-containing lipoprotein can be enhanced.

In the step v), by using gel filtration or an ultracentrifugation method, unreacted photosensitive substances can be removed, and the photosensitive substance-containing lipoprotein can be purified.

(Photodynamic therapy preparation)

The present embodiment relates to a photodynamic therapy preparation comprising a photosensitive substance-containing lipoprotein. The photodynamic therapy comprises administering a photosensitive substance-containing lipoprotein to a subject who is affected with, for example, malignant tumor or age-related macular degeneration, and applying light irradiation to the lesion site. In the photodynamic therapy, by administering a photosensitive substance-containing lipoprotein to a subject who is affected with malignant tumor or age-related macular degeneration, the photosensitive substance-containing lipoprotein is selectively delivered to the lesion site (e.g., new blood vessels in tumor tissues or in the posterior eye segment that is the affected area of age-related macular degeneration). Thereafter, light irradiation (laser light irradiation) is applied to the lesion site to which the photosensitive substance-containing lipoprotein has been delivered. Thereby, reactive oxygen and heat are generated from the photosensitive substance contained in the photosensitive substance-containing lipoprotein, and they can damage the lesion site and can destroy the lesion tissues. Thus, the photodynamic therapy is a treatment that combines the delivery of the photosensitive substance-containing lipoprotein to the lesion site with application of light irradiation (laser light irradiation) to the lesion site.

When the photodynamic therapy preparation is used in, for example, the treatment of malignant tumor, the type of the malignant tumor is not particularly limited. Examples of the malignant tumor may include brain and nerve tumors, skin cancer, stomach cancer, lung cancer, liver cancer, lymphoma and leukemia, colon cancer, pancreatic cancer, anal and rectal cancer, esophageal cancer, uterine cancer, breast cancer, adrenal cancer, kidney cancer, renal pelvis and ureter cancer, bladder cancer, prostate cancer, urethral cancer, penile cancer, testicular cancer, bone and osteosarcoma, leiomyoma, rhabdomyoma, and mesothelioma. Cancer cells have new blood vessels, and in the new blood vessels, $\alpha_v\beta_3$ integrin, aminopeptidase A, aminopeptidase N (APN; also called CD13), or a vascular endothelial growth factor (VEGF) receptor, etc. is expressed. Accordingly, the neovascular endothelial cell-targeting peptide and aptamer comprised in the photosensitive substance-containing lipoprotein specifically bind to these molecules.

The photodynamic therapy preparation of the present embodiment is particularly preferably used for the treatment of age-related macular degeneration. That is, in a preferred embodiment, the photodynamic therapy preparation of the present embodiment is a formulation for the treatment of age-related macular degeneration. In this case, in the photodynamic therapy, by administering a photosensitive substance-containing lipoprotein to a subject who is affected with age-related macular degeneration, the photosensitive substance-containing lipoprotein is selectively delivered to new blood vessels in the posterior eye segment that is the area affected by age-related macular degeneration. Thereafter, light irradiation (laser light irradiation) is applied to the new blood vessel generation site where the photosensitive substance-containing lipoprotein has reached. Thereby, reactive oxygen is generated from the photosensitive substance contained in the photosensitive substance-containing lipoprotein, and it can damage the new blood vessels and can occlude the blood vessels.

Moreover, the photodynamic therapy preparation of the present embodiment can also be used for the treatment of malignant tumors. In a preferred embodiment, the photodynamic therapy preparation of the present embodiment is a formulation for the treatment of malignant tumors. In this case, in the photodynamic therapy, by administering a photosensitive substance-containing lipoprotein to a subject having malignant tumor, the photosensitive substance-containing lipoprotein is selectively delivered to new blood vessels resulting from the malignant tumor. Thereafter, light irradiation (laser light irradiation) is applied to the new blood vessel generation site where the photosensitive substance-containing lipoprotein has reached. Thereby, reactive oxygen is generated from the photosensitive substance contained in the photosensitive substance-containing lipoprotein, and it can damage the new blood vessels and can occlude the blood vessels.

In addition to the aforementioned photosensitive substance-containing lipoprotein, the photodynamic therapy preparation may further comprise pharmaceutically acceptable additives. The pharmaceutically acceptable additives may include various active ingredients or medicinal ingredients (including pharmacologically active ingredients and physiologically active ingredients) and additives, depending on various types of intended uses, as long as they do not impair the effects of the present invention. Examples of such additives may include buffering agents (e.g., sodium phosphate), isotonicity agents (e.g., sodium chloride), pH adjusters (e.g., boric acid), antiseptics/preservatives (e.g., benzalkonium chloride), stabilizers (e.g., mannitol), thickeners (e.g., sodium alginate), chelating agents (e.g., sodium edetate), surfactants (e.g., polyoxyethylene sorbitan monooleate), absorption enhancers (e.g., sodium oleate), and flavors (e.g., menthol).

Furthermore, in addition to the aforementioned photosensitive substance-containing lipoprotein, the photodynamic therapy preparation may further comprise diagnostic, preventive or therapeutic agents for malignant tumor or age-related macular degeneration. Examples of such diagnostic, preventive or therapeutic agents may include vascular endothelial growth factor (VEGF) inhibitors (e.g., VEGF antibodies, VEGF aptamers, and siRNA), steroid preparations (e.g., dexamethasone, betamethasone, fluorometholone, and prednisolone), non-steroid preparations (e.g., indomethacin, bromfenac, diclofenac sodium, etc.), prostaglandin preparations (e.g., latanoprost and tafluprost), pirenoxine, glutathione, memantine, epinephrine, pilocarpine hydrochloride, carbachol, dorzolamide hydrochloride, acetazolamide, timolol maleate, carteolol hydrochloride, betaxolol hydrochloride, bunazosin hydrochloride, isopropyl unoprostone, pranoprafen, aspirin, SRPIN803 (6-(4-Hydroxy-3-methoxybenzylidene)-5-imino-2-(trifluoromethyl)-5H-[1,3,4]thiadiazolo[3,2-a]pyrimidin-7(6H)-one), and pazopanib.

The photodynamic therapy preparation of the present embodiment can be used in dosage forms such as eye drops, eye ointment, nasal drops, ear drops and injections, and among these, injections are preferable.

The photodynamic therapy preparation of the present embodiment is preferably an injection, and the photodynamic therapy preparation is preferably administered via intravenous injection. When a photodynamic therapy preparation is intravenously injected, the photosensitive substance-containing lipoprotein comprised in the photodynamic therapy preparation selectively accumulates at the lesion site. Thereafter, by applying a laser light to the lesion site where the photosensitive substance has accumulated, reactive oxygen and heat are generated from the photosensitive substance, and then, they damage the lesion site. Thereby, progression of the disease can be stopped. For example, in a case where the photodynamic therapy preparation is used for the treatment of age-related macular degeneration, when the photodynamic therapy preparation is intravenously injected, the photosensitive substance-containing lipoprotein comprised in the photodynamic therapy preparation selectively accumulates in new blood vessels in the posterior eye segment, which is the lesion site of age-related macular degeneration. Thereafter, by applying a laser light to the lesion site where the photosensitive substance has accumulated, reactive oxygen and heat are generated from the photosensitive substance, and then, they damage the new blood vessels and occlude the blood vessels. Moreover, in a case where the photodynamic therapy preparation is used for the treatment of malignant tumor, when the photodynamic therapy preparation is intravenously injected, the photosensitive substance-containing lipoprotein comprised in the photodynamic therapy preparation selectively accumulates in new blood vessels resulting from the malignant tumor. Thereafter, by applying a laser light to the lesion site where the photosensitive substance has accumulated, reactive oxygen and heat are generated from the photosensitive substance, and then, they damage the new blood vessels and occlude the blood vessels.

The photosensitive substance-containing lipoprotein comprised in the photodynamic therapy preparation of the present embodiment can selectively accumulate, in particular, in new blood vessels in the posterior eye segment, which is the area affected by age-related macular degeneration, and in new blood vessels resulting from malignant tumor. For this reason, the delivery efficiency of the photosensitive substance comprised in the photosensitive substance-containing lipoprotein can be increased. Furthermore, by increasing the selectivity of the delivery site of the photodynamic therapy preparation, it is possible to suppress the occurrence of phototoxicity (side effects) even in a case where a light is irradiated to sites other than the lesion site. Further, since the photodynamic therapy preparation of the present embodiment has a longer disease site retentivity than conventional drugs, it is possible to ensure a longer period of time during which a light can be irradiated. This makes it possible, for example, to administer the photodynamic therapy preparation outside of a laser light irradiation room, and the burden on medical professionals performing photodynamic therapy can be reduced and further, the therapeutic efficiency of the photodynamic therapy can be enhanced.

(Drug delivery method)

The present embodiment may relate to a method for delivering a drug to new blood vessels in the posterior eye segment, using the aforementioned photosensitive substance-containing lipoprotein. In addition, the present embodiment may also relate to a method for delivering a drug to new blood vessels in the posterior eye segment, using the aforementioned photodynamic therapy preparation.

Furthermore, the present embodiment may relate to a method for delivering a drug to new blood vessels in a malignant tumor part, using the aforementioned photosensitive substance-containing lipoprotein. In addition, the present embodiment may also relate to a method for delivering a drug to new blood vessels in a malignant tumor part, using the aforementioned photodynamic therapy preparation.

As mentioned above, the modified high-density lipoprotein (vcHDL) comprising a modified apolipoprotein and a phospholipid serves as a drug delivery carrier to the posterior eye segment or a drug delivery carrier to a malignant tumor part because the apolipoprotein contains VTP in addition to CP. Thus, the modified high-density lipoprotein can selectively deliver the photosensitive substance to new blood vessels. Therefore, by applying the drug delivery method to, or by the method of administering the above-mentioned photodynamic therapy preparation to, patients who are affected with or are at risk of having age-related macular degeneration, it becomes possible to diagnose, prevent, or treat the age-related macular degeneration. Moreover, by applying the drug delivery method to, or by the method of administering the above-mentioned photodynamic therapy preparation to, patients who have malignant tumor, it becomes possible to treat cancer, such as shrinking or eliminating malignant tumor or suppressing metastasis.

In the drug delivery method of the present embodiment, by using the modified high-density lipoprotein (vcHDL) comprising a modified apolipoprotein and a phospholipid as a drug delivery carrier, a photosensitive substance (drug) can be selectively delivered to new blood vessels in the posterior eye segment, the area affected by age-related macular degeneration, which seems to be caused by abnormal neovascularization. In addition, in the drug delivery method of the present embodiment, a photosensitive substance (drug) can be selectively delivered to new blood vessels resulting from a malignant tumor. Thereby, the delivery efficiency of the photosensitive substance (drug) can be increased. Furthermore, by increasing the selectivity of the drug delivery site, it is possible to suppress the occurrence of phototoxicity (side effects) even in a case where a light is irradiated to areas other than the lesion site. This allows for a wide range of light irradiation sites, it becomes possible to significantly simplify the treatment of age-related macular degeneration and cancer treatment. Further, by increasing the disease site retentivity of the drug, the time limit (15 minutes) between intravenous administration and laser light irradiation cannot only be relaxed, but the clearance of the photosensitive compound from normal tissue can also be promoted, thereby reducing side effects.

In the present embodiment, the modified high-density lipoprotein (vcHDL) itself, which does not contain a photosensitive substance (drug), can also exert anti-inflammatory action and neovascularization inhibitory action. Therefore, the above-described drug delivery method itself could become a method for treating or preventing age-related macular degeneration and cancer.

(Method for occluding new blood vessels in the posterior eye segment)

The present embodiment relates to a method for occluding new blood vessels in the posterior eye segment, wherein the method comprises administering the aforementioned photosensitive substance-containing lipoprotein to a subject who is affected with age-related macular degeneration, and applying light irradiation to a new blood vessel generation site in the posterior eye segment. It is to be noted that the method for occluding new blood vessels in the posterior eye segment can be applied to humans or mammals other than humans, but may exclude methods for treating humans in some cases. In the method for occluding new blood vessels in the posterior eye segment, by administering the aforementioned photosensitive substance-containing lipoprotein or photodynamic therapy preparation, for example, via intravenous injection, etc., the photosensitive substance-containing lipoprotein reaches the new blood vessel generation site in the posterior eye segment. Since the photosensitive substance-containing lipoprotein comprises a modified apolipoprotein containing VTP and CP, the photosensitive substance-containing lipoprotein is selectively delivered to new blood vessels in the posterior eye segment, the area affected by age-related macular degeneration. Thereafter, light irradiation (laser light irradiation) is applied to the new blood vessel generation site where the photosensitive substance-containing lipoprotein has reached. Thereby, reactive oxygen is generated from the photosensitive substance contained in the photosensitive substance-containing lipoprotein, and it damages the new blood vessels and occludes the blood vessels.

Moreover, the present embodiment relates to a method for occluding new blood vessels in a malignant tumor par, wherein the method comprises administering the aforementioned photosensitive substance-containing lipoprotein to a subject having malignant tumor, and applying light irradiation to a new blood vessel generation site in the malignant tumor part. It is to be noted that the method for occluding new blood vessels in a malignant tumor part can be applied to humans or mammals other than humans, but may exclude methods for treating humans in some cases. In the method for occluding new blood vessels in a malignant tumor part, by administering the aforementioned photosensitive substance-containing lipoprotein or photodynamic therapy preparation, for example, via intravenous injection, etc., the photosensitive substance-containing lipoprotein reaches the new blood vessel generation site in the malignant tumor part. Since the photosensitive substance-containing lipoprotein comprises a modified apolipoprotein containing VTP and CP, the photosensitive substance-containing lipoprotein is selectively delivered to new blood vessels resulting from malignant tumor. Thereafter, light irradiation (laser light irradiation) is applied to the new blood vessel generation site where the photosensitive substance-containing lipoprotein has reached. Thereby, reactive oxygen is generated from the photosensitive substance contained in the photosensitive substance-containing lipoprotein, and it damages the new blood vessels and occludes the blood vessels. Thereby, it becomes possible to treat cancer, such as shrinking or eliminating malignant tumor or suppressing metastasis.

The light to be irradiated can be, for example, a light emitted by semiconductor lasers or dye lasers, etc. Specific examples may include a Nd-YAG (yttrium aluminum garnet) laser, a $CO_2$ laser, an argon dye laser, and an excimer laser. For example, light irradiation (laser light irradiation) can be performed in accordance with the treatment guidelines disclosed in Non-Patent Document 1, etc. In this case, the wavelength of the laser light is 689 $\pm$ 3 (mean $\pm$ standard deviation) nm, and the light irradiation energy is set as 50 J/(light irradiation output of 600 mW/cm$^2$ for 83 seconds).

It is to be noted that, when the photosensitive substance-containing lipoprotein or photodynamic therapy preparation of the present embodiment is intravenously injected, its retention time in blood is relatively long. Accordingly, the time period for the light irradiation (laser light irradiation) can be set to, for example, approximately 5 to 80 minutes after initiation of intravenous injection of the photosensitive substance-containing lipoprotein or photodynamic therapy preparation. When a conventional photodynamic therapy preparation is used, the time period for the light irradiation (laser light irradiation) is approximately 5 to 20 minutes after initiation of intravenous injection of the photosensitive substance-containing lipoprotein or photodynamic therapy preparation. Therefore, by using the photosensitive substance-containing lipoprotein or photodynamic therapy preparation of the present embodiment, it becomes possible to significantly extend the time period for the light irradiation (laser light irradiation).

When age-related macular degeneration or cancer is treated, it is preferable to perform the method for occluding new blood vessels in the posterior eye segment one or multiple times.

Examples of other forms of the present embodiment may include the following. It is to be noted that, regarding the treatment method, it may be applied to humans or non-human mammals, but may exclude methods for treating humans in some cases.

[A] The aforementioned photosensitive substance-containing lipoprotein, which is for use in photodynamic therapy.

[B] Use of the aforementioned photosensitive substance-containing lipoprotein for production of a photodynamic therapy preparation.

[C] The aforementioned photosensitive substance-containing lipoprotein, which is for use in the treatment of age-related macular degeneration.

[D] Use of the aforementioned photosensitive substance-containing lipoprotein for production of a preparation for the treatment of age-related macular degeneration.

[E] The aforementioned photosensitive substance-containing lipoprotein, which is for use in the treatment of malignant tumor.

[F] Use of the aforementioned photosensitive substance-containing lipoprotein for production of a preparation for the treatment of malignant tumor.

[G] The aforementioned photosensitive substance-containing lipoprotein, which is for use in a method for occluding new blood vessels in the posterior eye segment.

[F] The aforementioned photosensitive substance-containing lipoprotein, which is for use in a method for occluding new blood vessels in a malignant tumor part.

Examples

The features of the present invention will be more specifically described in the examples and comparative examples described below. In the following examples, the materials, the amounts used, ratios, the details of the treatments, the treatment procedures, etc. may be modified, as appropriate, unless they overstep the spirit and the scope of the invention. Accordingly, the invention should not be restrictively interpreted by the following specific examples.

(Example 1)

Preparation of NGR- and PEN-fused apoA-I and PEN-fused apoA-I

The structure of NGR- and penetratin (PEN)-fused apolipoprotein A-I (apoA-I) is as shown below.

Human apoA-I with 44 amino acids deleted at the N-terminus was used as a template, and the NGR (CNGRCGG) moiety was allowed to bind to the N-terminus of the N-terminal 44-amino acid-deleted human apoA-I. Furthermore, RQIKIWFQNRRMKWKKK (SEQ ID NO: 1), which had K added to the C-terminus of PEN, was allowed to bind to the C-terminus of the N-terminal 44-amino acid-deleted human apoA-I. The molecular weight of the NGR- and PEN-fused apoA-I was approximately 29,319 Da.

Moreover, human apoA-I with 44 amino acids deleted at the N-terminus was used as a template, and RQIKIWFQNRRMKWKKK (SEQ ID NO: 1), which had K added to the C-terminus of PEN, was allowed to bind to the C-terminus of the N-terminal 44-amino acid deleted human apoA-I, to obtain PEN-fused apoA-I.

Hereafter, NGR- and PEN-fused apoA-I and PEN-fused apoA-I are also collectively referred to as apoA-I variants.

The ApoA-I variants were allowed to express in BL21 (DE3) (Novagen, Dallas, WI, USA), and were then purified under guanidine denaturing conditions using Ni Sepharose High-Performance resin filled into a Tricorn 10/100 column of an AKTAstart chromatography system (Cytiva, Tokyo, Japan).

Preparation of modified high-density lipoprotein

Sodium cholate was mixed into distearoyl phosphatidylcholine (DSPC) at a molar ratio of 1 : 3.6, and the DSPC was dispersed by bath sonication (Elma Schmidbauer GmbH, Mannheim, Germany) at 65°C for 10 min. Subsequently, each apoA-I variant was dissolved in PBS containing 4 M urea, and was mixed with the DSPC dispersion (DSPC : apoA-I variant = 80 : 1 (mol)). The mixture was then subjected to a static reaction at 55°C for 30 min. After completion of the reaction, the reaction mixture was dialyzed overnight at room temperature against 3 L of PBS. For the dialysis, a dialysis membrane with a molecular weight cutoff of 50 kDa (Spectrum Chemical Manufacturing Corp.) was used. It is to be noted that only the mixed solution of DSPC and NGR- and PEN-fused apoA-I was allowed to cool until room temperature, after starting the dialysis at 55°C. The reaction solution inside the dialysis membrane was transferred into a microtube and was then centrifuged (20,000 x g, 24°C, 30 min). Thereafter, the supernatant was collected, and was then purified by size-exclusion chromatography using a HPLC (Shimadzu Corporation, Kyoto, Japan) connected with a Superose 6 prep grade packed column (Cytiva). A modified high-density lipoprotein (vcHDL) comprising each apoA-I variant (hereafter, the HDL variant using PEN-fused apoA-I is referred to as "eLP1," and the HDL variant using NGR- and PEN-fused apoA-I is referred to as "eLP2") was eluted using a tris(hydroxymethyl)aminomethane buffer (150 mM NaCl, 50 mM Tris·HCl (pH 7.4), 2 mM ethylenediaminetetraacetic acid, and 0.5 mM phenylmethylsulfonyl fluoride) at a flow rate of 1 mL/min at room temperature. The eluate of the modified HDL fraction (50-75 min) was collected, and was then dialyzed against 3 L of PBS, using a dialysis membrane with a molecular weight cutoff of 50 kDa overnight at room temperature. The modified HDL inside the dialysis membrane was transferred into a microtube, and was then centrifuged (20,000 x g, 24°C, 15 min). The supernatant was stored in a protein low adsorption tube.

Loading of verteporfin (VP) onto modified high-density lipoprotein

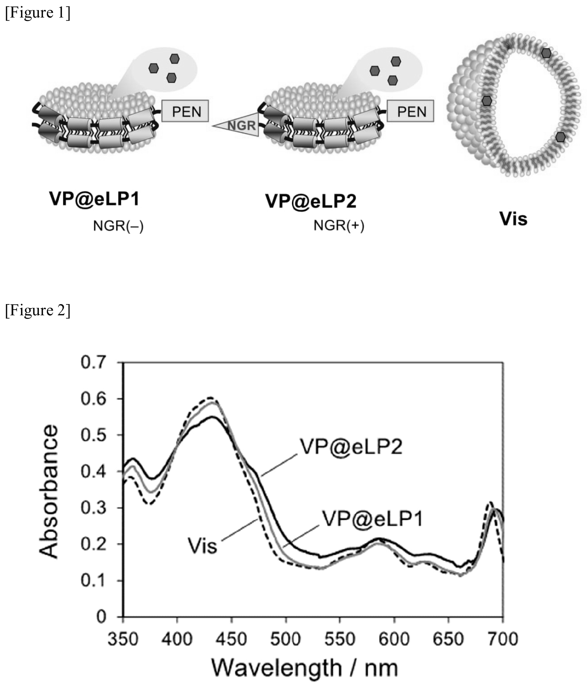

Modified HDL was mixed with VP dissolved in DMSO, to result in a VP/lipid molar ratio of 1 : 10, and the mixture was then subjected to a static reaction at 55°C for 30 min. Thereafter, purification was performed by size-exclusion chromatography equilibrated with PBS. VP-loaded modified high-density lipoproteins (VP@eLP1, VP@eLP2) were stored in protein low-adsorption tubes (Figure 1).

(Example 2)

Preparation of Visudyne®-like nanoparticles (Vis)

DMPC dissolved in ethanol, egg PG, and VP dissolved in methanol were mixed with one another at a molar ratio of 40 : 24 : 5. The ethanol was evaporated under reduced pressure at 35°C using a rotary evaporator R-300 (Nihon BUCHI, Tokyo, Japan) to form a lipid membrane. Subsequently, the resulting lipid membrane was freeze-dried for 2 hours in a freeze-drying machine FDS-1000 (TOKYO RIKAKIKAI Co., Ltd., Tokyo, Japan). To this lipid membrane, a 9.9% lactose solution was added, and the resulting membrane was subjected to bath sonication for 10 minutes at room temperature. After the sonication, the lipid suspension was passed through a polycarbonate filter (Avanti Polar Lipids) having a pore size of 400 nm, using an extruder Mini-Extruder (Avanti Polar Lipids, Alabama, USA) to form Vis (Figure 1).

(Evaluation)

Evaluation of properties of VP-loaded modified high-density lipoprotein and Vis

The protein concentration contained in the VP-loaded modified high-density lipoprotein was quantified. For the quantification, bovine serum albumin (Thermo Fisher Scientific) was used as the standard solution, and a DC protein assay kit (Bio-Rad Laboratories, Inc.) and a Synergy HTX Multi-Mode Microplate Reader (BioTek Instruments, Inc., Winooski, VT, USA) were employed in the coexistence of 1% TritonX-100. The concentration of phosphatidylcholine contained in the VP-loaded modified high-density lipoprotein and in Vis was quantified using a Phospholipid C assay kit (FUJIFILM Wako Pure Chemical Corporation). The volume average particle diameter and surface charge of each nanoparticle were measured using a Nanotrac UPA-UT151 particle size analyzer (MicrotracBEL Corp., Tokyo, Japan) and a ZETASIZER NANO Z (Malvern, Worcestershire, U.K.), respectively. The VP concentration in each nanoparticle produced was calculated from the integrated fluorescence intensity in the fluorescence spectrum using a plate reader (TECAN Group Ltd., Männedorf, Switzerland). In addition, the absorption spectrum of each nanoparticle was measured using an ultraviolet-visible-near-infrared spectrophotometer UV-3600 Plus (Shimadzu Corporation, Kyoto, Japan) (Figure 2). As shown in Figure 2, almost no difference was found in the absorption peaks of the VP-loaded modified high-density lipoprotein and the Vis.

[Table 2]

|  | VP@eLP1 | VP@eLP2 | Vis |
|---|---|---|---|
| VP/lipid (mol/mol) | 0.08 ± 0.02 | 0.09 ± 0.03 | 0.11 ± 0.04 |
| Average particle diameter (nm) | 13 ± 0.04 | 19 ± 3 | 178 ± 64 |
| Surface charge (mV) | -12 ± 4 | -12 ± 4 | -72 ± 10 |

Singlet oxygengeneration ability

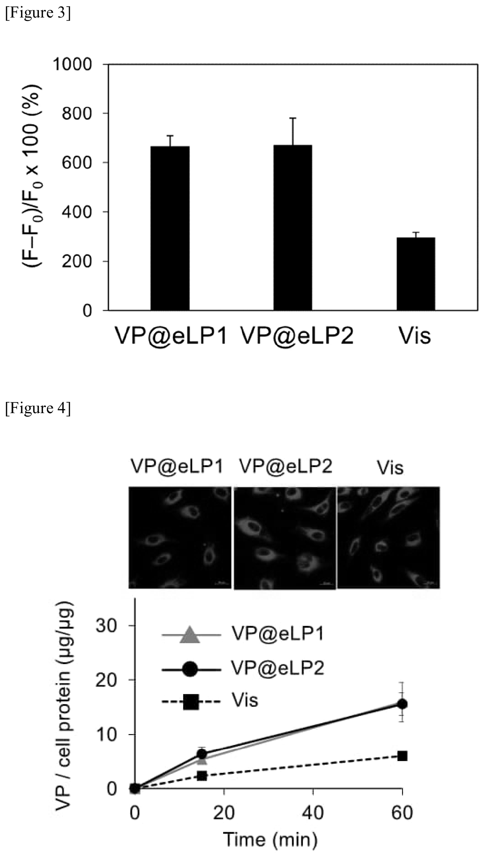

Singlet oxygen ($^1O_2$) was detected using Singlet Oxygen Sensor Green Reagent (SOSG; Invitrogen Thermo Fisher Scientific Inc., USA), which selectively reacts with $^1O_2$ and generates fluorescence. A solution comprising nanoparticles (VP@eLP1, VP@eLP2, or Vis) containing 10 µg/mL VP, and 10 mM SOSG, was prepared. Each nanoparticle dispersion was placed in a square cell, and was then irradiated (10 mW, 5 min) with a 689 nm xenon lamp (Asahi Spectra, Tokyo, Japan). The fluorescence spectra of the solution before and after the light irradiation were measured using a plate reader (TECAN) (Ex. 480 nm/Em. 500 to 560 nm), and the integrated fluorescence intensity was calculated. The integrated fluorescence intensity before the light irradiation was defined as $F_0$, and the integrated

fluorescence intensity after the light irradiation was defined as F. The $^1O_2$ generation ability was calculated using the expression shown below. The calculation results of the $^1O_2$ generation ability are shown in Figure 3. The reason why the $^1O_2$ generation ability of Vis is smaller than those of VP@eLP1 and VP@eLP2 is considered to be because the lipids that constitute Vis have unsaturated fatty acids, which react with $^1O_2$ and are oxidized.

[Expression 2]

$$(F - F_0) / F_0 \times 100 \ (\%)$$

Cell culture

Using a 100-mm dish, HUVECs were cultured in a medium containing endothelial cell growth basal medium-2 (EBM2), 2% fetal bovine serum, and 1% antibiotic antimycotic mixed stock solution (Nacalai Tesque, Inc.), in a HERAcell 150 (Heraeus Holding GmbH, Hanau, Germany) incubator (37°C, 5% $CO_2$). The medium was exchanged every two days. The cells were sub-cultured, after confirming that individual cells reached 70 to 90% confluence. The cells were detached by treating them with TrypLE Express (Thermo Fisher Scientific). The individual cells detached were centrifuged (200 x g, 24°C, 3 min), the supernatant then removed, and the residue was then suspended in a fresh medium, which was then transferred to a new dish, to carry out the subculture.

Analysis of interaction of each nanoparticle with vascular endothelial cells

HUVECs were used as a model of normal vascular endothelial cells. The culture was performed in EBM2 containing 2% FBS. Basic fibroblast growth factor (bFGF) was added to the medium to a final concentration of 50 ng/mL in order to enhance CD13 expression. HUVECs were seeded at a density of 1 x $10^5$ cells/mL in a triple-well glass-based dish, and were then subjected to a static culture for 24 hours in a $CO_2$ incubator at 37°C. Each nanoparticle (eLP1, eLP2, or Vis, each containing 1 $\mu$g/mL VP) was allowed to act on adherent cells, and after 15 minutes and 60 minutes, the cells were washed with D-PBS containing heparin, and intracellular VP fluorescence was then observed using a confocal fluorescence microscope LSM800 with Airyscan (Carl Zeiss Microscopy GmbH, Jena, Germany). For the observation, a 40x oil immersion objective lens (Plan-Apochromat 40x/1.4 oil DIC M27) was used.

For quantification of intracellular VP amount, HUVECs were seeded in a 12-well plates (1 x $10^5$ cells/mL) and were used. As mentioned above, each nanoparticle was allowed to act on the cells, and thereafter, the cells were detached using a cell scraper, and were then centrifuged (1,000 x g, 4°C, 5 min) to obtain a cell pellet. The obtained pellet was dissolved in 400 $\mu$L of RIPA buffer (25 mM Tris·HCl (pH 7.6), 0.1% SDS, 150 mM NaCl, 1% Nonidet P-40, and 1% sodium deoxycholate), and was then subjected to two freeze-thaw cycles (at -80°C for 30 min; and at 37°C for 30 min) repeatedly. The concentration of the protein in the supernatant obtained by centrifugation (12,000 x g, 4°C, 10 min) was quantified using a BCA protein assay kit, and the concentration of VP was quantified by fluorescence spectrum measurement using a plate reader. The intracellular VP weight was calculated by normalizing the VP weight by the cell protein weight (Figure 4). VP@eLP2 showed a VP cellular uptake equivalent to that of Vis.

Phototherapeutic effect of each nanoparticle

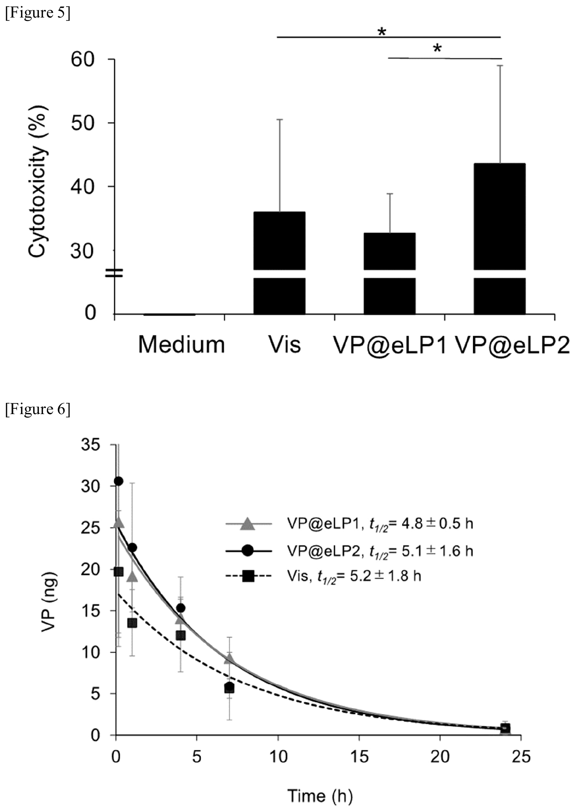

A photocytotoxicity test for each nanoparticle was carried out using the Cytotoxicity LDH Assay Kit-WST (DOJINDO LABORATORIES, Kumamoto, Japan). HUVECs were seeded in a 96-well plate at a density of 1 x $10^5$ cells/mL, and were then stimulated with bFGF by the same method as that described above. Thereafter, each nanoparticle containing 100 ng/mL VP was allowed to act on the cells. Sixty minutes later, the cells were washed with D-PBS containing heparin, the resulting cells were then newly placed in a medium, and light irradiation was then carried out using a xenon lamp (31 mW, 1 min). After completion of the light irradiation, the medium was replaced with a fresh medium, and the cells were then subjected to a static culture in a $CO_2$ incubator at 37°C for 24 hours. Cytotoxicity was evaluated according to the reagent manufacturer's protocols (Figure 5). As shown in Figure 5, P@eLP2 exhibited the highest cytotoxicity (= phototherapeutic effect).

Retentivity of each nanoparticle in blood

The animal experiments using mice, which were conducted in the present study, were carried out in accordance with the ethical guidelines established by the Association for Research in Vision and Ophthalmology Statement for the Use of Animals in Ophthalmic and Vision Research, the Kyoto University Animal Experimentation Guidelines, and the Toyama Prefectural University Animal Experimentation Ethics Committee. C57BL/6JJmsSlc mice (male, 8 to 10 weeks old) were purchased from Sankyo Labo Service Corporation, Inc. (Toyama, Japan). Each nanoparticle was administered into the tail vein of the mouse at a dose of 2 mgVP/kg body weight. Blood was collected 0.16, 1, 4, 7, and 24 hours after the administration, and was then mixed with 3.8% sodium citrate (Iwaki Seiyaku Co., Ltd., Tokyo, Japan) contained in a tube. After centrifugation (3000 rpm, 4°C, 5 min) had been performed to separate the plasma component from the blood cell component, the plasma as a supernatant component was collected, and was pre-frozen at -80°C, followed by freeze-drying. Methanol was added to all freeze-dried plasma component samples, followed by vortex mixing. The obtained samples were subjected to bath sonication for 2 minutes, and was then vortex-mixed again. The samples were stirred using a tube rotator (4°C, 1 h). Bath sonication was again performed for 2 minutes, followed by vortex mixing. After centrifugation (20,000 x g, 4°C, 5 min), the supernatant was analyzed by reverse phase high-performance liquid chromatography (RP-HPLC), and VP was quantified. VP was eluted using an HPLC LC-20Ai (Shimadzu Corporation) equipped with a liquid delivery unit (LC-20AD), an autosampler (SIL-20A), a column oven (CTO-20A), a photodiode array detector (SPD-M20A), and a spectrofluorometric detector (RF-20A xs). A COSMOSIL 5C18-MS-II column was used, and elution was performed using a gradient mobile phase consisting of (A) a 0.05 mM $NaH_2PO_4$ solution and (B) MeOH. The gradient program was set as follows: 0 to 2 min, 15% mobile phase B; 2 to 5 min, 15%-85% mobile phase B; and 5-10 min, 5%-100% mobile phase B. The flow rate was set at 1.1 mL/min, and the column oven temperature was set at 35°C (Figure 6). As shown in Figure 6, the $t_{1/2}$ of Vis was 5.2 $\pm$ 1.8 h, the $t_{1/2}$ of VP@eLP1 was 4.8 $\pm$ 0.5 h, and the $t_{1/2}$ of VP@eLP2 was 5.1 $\pm$ 1.6 h, indicating that no significant difference was found in blood retentivity.

Evaluation of CNV accumulation of VP loaded on each nanoparticle

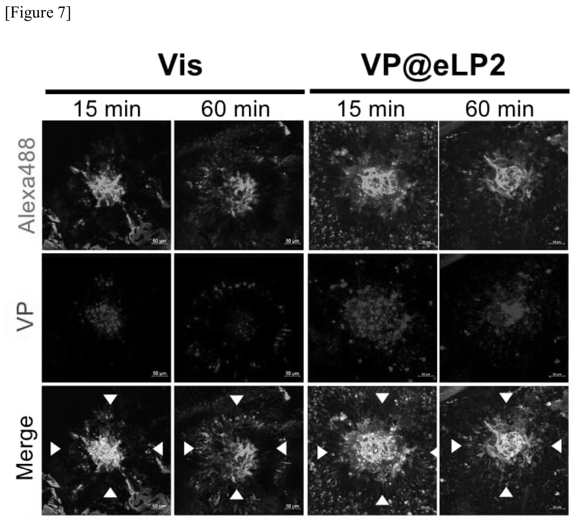

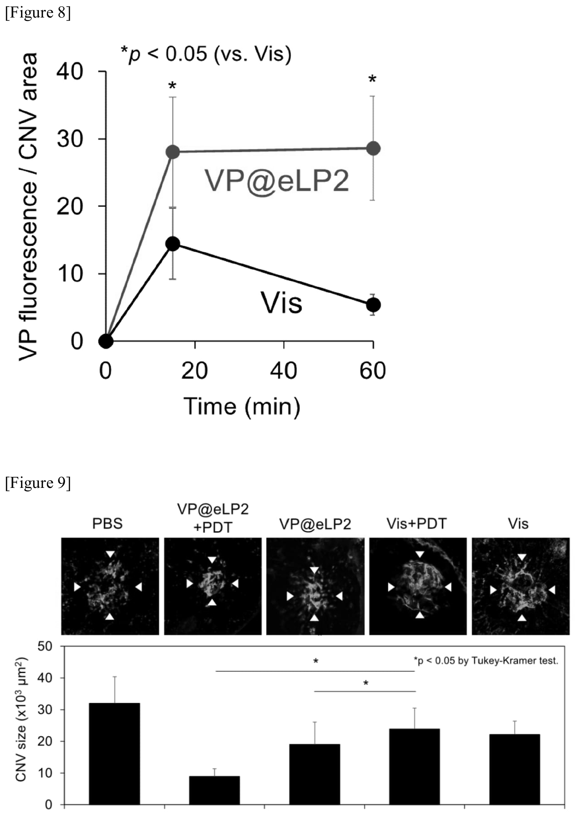

C57BL/6JJmsSlc mice (male, 8 to 10 weeks old) were used. The mice were anesthetized with 10 mg/kg xylazine and 80 mg/kg ketamine. Pupils were sufficiently dilated with 5% phenylephrine hydrochloride/0.8% tropicamide (Sandol P, Rhoto Nitten Co., Ltd, Nagoya, Japan). Cover glasses were placed on the mouse corneas, and were used as contact lenses. Laser light coagulation was performed using a photocoagulator (DC-3000; Nidek, Gamagori, Japan) that was set at 75 lm, 0.05 seconds, and 150 mW. CNV was generated by irradiating four areas 4 to 5 disc diameters apart from the optic disc with a laser light. Seven days after the laser light irradiation, each nanoparticles (Vis and VP@eLP2) were administered via the tail vein to the mice at a dose of 10 mg/kg converted to VP. At 15 and 60 minutes after the administration, the eyeballs were enucleated. After the enucleation, the eyeballs were fixed in 4% PFA, were then blocked, and were then immunostained using a rat anti-CD102 antibody (1 : 200, BD Pharmingen, San Diego, CA, USA) and an Alexa 488-labeled anti-rat IgG antibody (1 : 500, Invitrogen, Eugene, OR, USA) as primary and secondary antibodies, respectively. Thereafter, the RPE/choroid complex was flattened and was radially incised to obtain flat mounts. Fluorescence derived from the CNV (Ex. 488 nm/Em. 495 to 530 nm) and fluorescence derived from the VP within the CNV (Ex. 405 nm/Em. 610 to 700 nm) were observed by confocal microscopy (Figure 7). For the observation, a 10x dry objective lens (Plan-Apochromat 20x/0.8) was used. The fluorescence intensity of the VP within the CNV was quantified and analyzed using ImageJ (Figure 8). As shown in Figure 7, VP@eLP2 showed higher accumulation in new blood vessels in the choroid (choroidal neovascularization; CNV) than Vis. Furthermore, as shown in Figure 8, VP@eLP2 also showed significantly higher CNV retentivity than Vis.

Photodynamic therapy (PDT) effect on CNV mice

CNV was generated by the same method as that for evaluation of CNV accumulation. Seven days after laser light irradiation, mice were injected with Vis and VP@eLP2 via the tail vein at a dose of 2 mg/kg converted to VP. In addition, as a control, PBS was administered to the mice via the tail vein. Sixty minutes after the administration, a laser light irradiation was carried out. A laser light was applied to the entire eyeball using a Vislas PDT System 690S (Carl Zeiss) at a setting of 7000 $\mu m^2$, 60 s, and 600 mW/$cm^2$. Seven days after the irradiation, the eyes were enucleated, were then fixed in 4% PFA, were then blocked, and were then immunostained using a rat anti-CD102 antibody and an Alexa 488-labeled anti-rat IgG antibody as primary and secondary antibodies, respectively. Thereafter, the RPE/choroid complex was flattened and was radially incised to obtain flat mounts. Fluorescence derived from the CNV was observed by confocal microscopy. For the observation, a 20x dry objective lens (Plan-Apochromat 20x/0.8) was used. The CNV area value was quantified and analyzed (Figure 9). As shown in Figure 9, laser light irradiation reduced the CNV area in VP@eLP2-administered mice, compared with Vis-administered mice, demonstrating that a high phototherapeutic effect was obtained. Moreover, VP@eLP2-admnisterd mice showed a decrease in the CNV area even in a case where laser light irradiation was not applied, and this is considered because VP@eLP2 itself exerted anti-inflammatory action and neovascularization inhibitory action.

Statistical analysis

Statistical analysis was performed using Microsoft Excel. Differences between mean values of two or more groups were determined by Welch's t-test. Differences between mean values of three or more groups were determined by Tukey or Tukey-Kramer test. For all statistical analyses, $p < 0.05$ was considered to be statistically significant. All data were graphed as mean $\pm$ standard deviation.

Consideration

As shown in the above-described results, VP@eLP2 exhibits higher CNV selectivity and significantly higher CNV retentivity than Vis. The high CNV selectivity of VP@eLP2 makes it possible to reduce side effects (phototoxicity). Moreover, the high CNV retentivity of VP@eLP2 makes it possible to extend the PDT-possible time range. These effects may lead to the development of photodynamic therapy that is less burdensome for both medical professionals and patients.

Phototoxicity (side effects) test

In order to confirm that phototoxicity (side effects) is reduced when VP@eLP2 is used, the following test was carried out.

Vis and VP@eLP2, at a dose of 2 mg/kg converted to VP, were administered via the tail vein to normal C57BL/6JJmsSlc mice (male, 8 to 10 weeks old). In addition, as a control, PBS was administered to the mice via the tail vein. Fifteen minutes after the administration, a laser light irradiation was carried out. A laser light was applied to the entire eyeball using a Vislas PDT System 690S (Carl Zeiss) at a setting of 7000 $\mu m^2$, 60 s, and 600 mW/cm$^2$. Seven days after the irradiation, the eyes were enucleated, were then fixed (4°C, overnight) in 4% paraformaldehyde (PFA, Nacalai Tesque, Inc.) and were then dehydrated in a 20% sucrose solution (4°C, 24 h). After the dehydration, the eyeballs were embedded in OCT compound (Miles, Elkhart, IN, USA), and were then frozen in liquid nitrogen. The frozen eyeballs were sectioned at 15 $\mu m$ using a cryostat microtome (Leica Microsystems, Tokyo, Japan). The prepared sections were stained by H&E staining, and were then mounted using Aqua-Poly/Mount (Polysciences, Inc., USA). The retina margin portions of the H&E-stained sections were observed using an OLYMPUS IXplore Pro (Olympus Corporation, Tokyo, Japan).

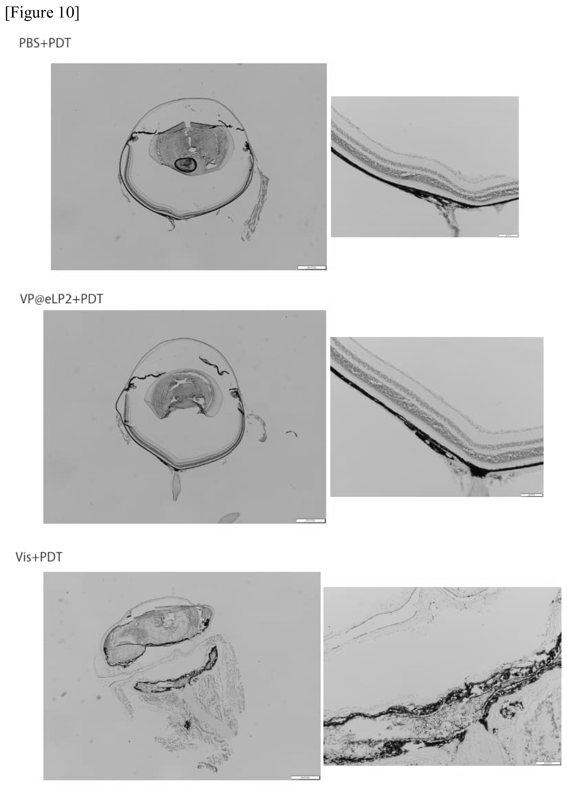

As shown in Figure 10, when the mice administered with PBS and VP@eLP2 were irradiated with a laser light, no abnormalities were observed in the retina and no phototoxicity occurred. On the other hand, when the mice administered with Vis were irradiated with a laser light, the entire retina disappeared. and deformation of the eyeball occurred. This is considered to be due to the low CNV selectivity of Vis, which resulted in damage (phototoxicity) to the retina caused by the laser light irradiation.

VP@eLP2 uptake by HUVECs in presence of CD13 inhibitor

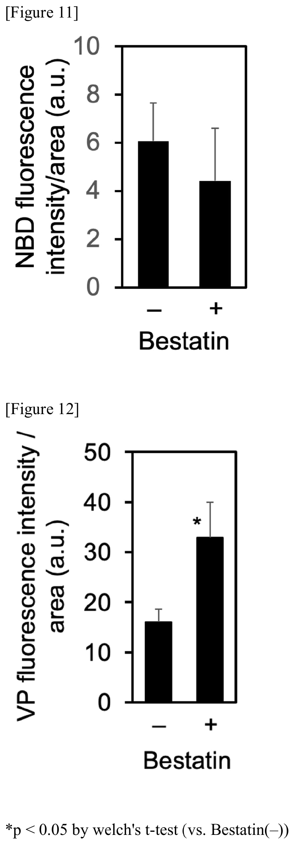

HUVECs were used as a model of normal vascular endothelial cells. The culture was performed in EBM2 containing 2% FBS. Basic fibroblast growth factor (bFGF) was added to the medium to a final concentration of 50 ng/mL in order to enhance CD13 expression. Further, as a CD13 inhibitor, 250 $\mu$M bestatin (Sigma-Aldrich, St. Louis, MO) was added. HUVECs were seeded at a density of 1 x 10$^5$ cells/mL in a triple-well glass-based dish, and were then subjected to a static culture for 24 hours in a CO$_2$ incubator at 37°C. Thereafter, VP@eLP2 containing 1 $\mu$g/mL VP and a fluorescent lipid (NBD) was allowed to act on adherent cells, and after 60 minutes, the cells were washed with D-PBS containing heparin, and were then observed using a confocal fluorescence microscope LSM800 with Airyscan (Carl Zeiss Microscopy GmbH, Jena, Germany). The fluorescence intensity of VP and the fluorescence intensity of NBD in the cells were evaluated using ImageJ (Figure 11 (NBD fluorescence intensity) and Figure 12 (VP fluorescence intensity)).

Consideration

In the presence of bestatin, NBD fluorescence intensity did not change significantly. On the other hand, VP fluorescence intensity significantly increased. This difference may be explained by the function of CD13 to enhance the expression of drug efflux transporters (Guo et al., Oncol. Res. 2020, 28, 533-540). Assuming that VP is a better substrate for drug efflux transporters than NBD, it is considered that the influence of the inhibition of drug efflux transporter expression by bestatin has enhanced the intracellular retentivity of VP rather than the inhibition of VP@eLP2 cellular uptake. In other words, the NGR peptide appears to have a greater influence on drug retentivity in CD13-positive cells, rather than on drug targeting to CD13-positive cells.

Analysis of expression levels of various types of transporter mRNAs by quantitative reverse transcription-polymerase chain reaction (qRT-PCR)

HUVECs were seeded at a density of 1 x 10$^5$ cells/mL in 2 mL on a 6-well plate (medium composition: EBM2, 2% FBS, and 50 $\mu$g/mL bFGF). After 24 hours, the culture supernatant was removed, the cells were then washed with 1 mL of D-PBS, 2 mL of VP@eLP2 containing 100 $\mu$M VP (100 $\mu$M VP) was then added to the resulting cells, and the cells were left at rest at 37°C for 1 hour in a $CO_2$ incubator. Thereafter, the cells were washed with 1 mL of D-PBS, and the plate was immediately cooled on ice. Total RNA was extracted from the cells using ISOGEN II (Nippon Gene Co., Ltd., Tokyo). In order to obtain cDNA, RNA was reverse transcribed using SuperScript VILO Master Mix. Subsequently, Real-time PCR was performed using a Master Mix (TB Green Premix Ex TaqII [Tli RNase H Plus], TaKaRa Bio, Inc.) and a Thermal Cycler Dice Real Time System (TaKaRa Bio, Inc.). Relative changes in the gene expression were analyzed using a $2^{-\Delta\Delta Ct}$ method. Human glyceraldehyde-3-phosphate dehydrogenase (hGAPDH) was used as an endogenous normalization control. The primer sequences used for Real-time PCR are shown below.

Consideration

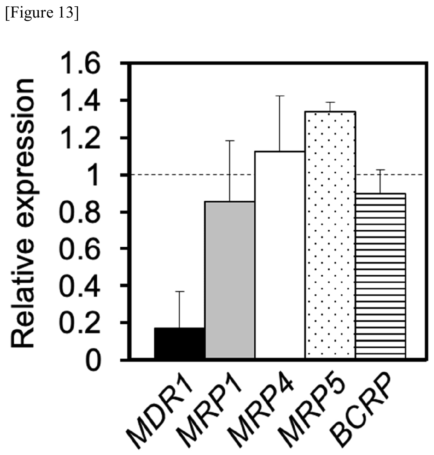

The expression of MDR1 mRNA was significantly reduced by the VP@eLP2 treatment (Figure 13). This result supports the above-described results and suggests that MDR1 is the drug efflux transporter responsible for VP efflux in vascular endothelial cells.

hMDR1

forward, 5'-AAGCTTAGTACCAAAGAGGCTCTG-3' (SEQ ID NO: 6)
reverse, 5'-GGCTAGAAACAATAGTGAAAACAA-3' (SEQ ID NO: 7)

hMRP1

forward, 5'-AGTGACCTCTGGTCCTTAAACAAGG-3' (SEQ ID NO: 8)
reverse, 5'-GAGGTAGAGAGCAAGGATGACTTGC-3' (SEQ ID NO: 9)

hMRP4

forward, 5'-TCCTCCTCCATTTACAGTGACA-3' (SEQ ID NO: 10)
reverse, 5'-TTATTCTCCTAAACACTGCAGCTC-3' (SEQ ID NO: 11)

hMRP5

forward, 5'-TGAATCTGAAGTGATGGAGAATGG-3' (SEQ ID NO: 12)
reverse, 5'-CCTATCGGAGCCTAGAACCG-3' (SEQ ID NO: 13)

hBCRP

forward, 5'-CCCAGTACGACTGTGACAATG-3' (SEQ ID NO: 14)
reverse, 5'-CACAGTCTTCAAGGAGATCAGCTA-3' (SEQ ID NO: 15)

hGAPDH

forward, 5'-ACGGATTTGGTCGTATTGGG-3' (SEQ ID NO: 16)
reverse, 5'-ACGGATTTGGTCGTATTGGG-3' (SEQ ID NO: 17)

PDT for cancer mouse models

The human prostate cancer cell line PC-3 (RIKEN Cell Bank) was used. A PC-3 suspension (4 x 10$^7$ cells/mL) in RPMI1640 was cooled on ice, and was then mixed with Corning Matrigel Matrix High Concentration (Corning, NY, USA) at a volume ratio of 1 : 1. Thereafter, 50 $\mu$L of this mixed solution was subcutaneously injected into the left and right dorsal regions of nude mice. Twelve days later (day 0), the major axis and minor axis of the subcutaneous tumors were measured with a vernier caliper, and the tumor volume was then calculated. Thereafter, VP@eLP2 or Vis (2 mg VP/kg) was intravenously administered to the tail vein of the mice. One hour later, under isoflurane anesthesia, a 690 nm laser was irradiated (815 mW/cm$^2$, $\varphi$5 mm, 2 min) on only one of the tumors on the left and right dorsal regions. Five days later (day 5), the major axis and minor axis of the subcutaneous tumors were measured with a vernier caliper, and the tumor volume was calculated according to the following equation:

$$\text{Tumor volume (cm}^3) = \text{minor axis (cm) x minor axis (cm) x major axis (cm)}/2$$

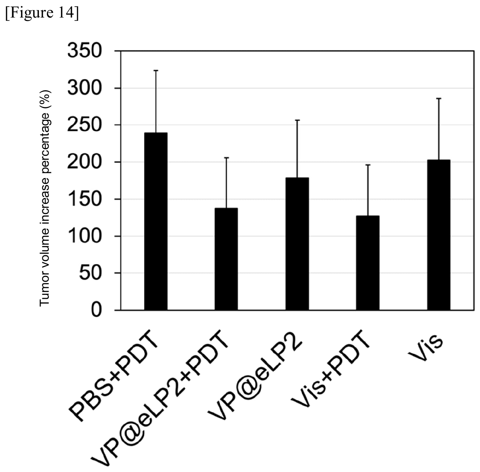

The effects of PDT were determined by normalizing the tumor volume on day 5 by the value on day 0 (Figure 14).

Consideration

In all groups, the tumor volume increased on day 5, compared to day 0. However, the tumor volume increase percentages in both the VP@eLP2 + PDT group and the Vis + PDT group were smaller than the values of the laser unirradiated groups (VP@eLP2 and Vis). Therefore, both samples are considered to exhibit the same level of PDT effects on tumor-bearing mice.

**Claims**

1. A photosensitive substance-containing lipoprotein, comprising a modified high-density lipoprotein containing an apolipoprotein and a phospholipid, and a photosensitive substance, wherein

   the photosensitive substance-containing lipoprotein comprises one or more molecules of the photosensitive substance per particle of the modified high-density lipoprotein, and
   the apolipoprotein is formed by binding between a vascular endothelial cell-targeting peptide or aptamer and a cell affinity peptide.

2. The photosensitive substance-containing lipoprotein according to claim 1, wherein the photosensitive substance is one type or two or more types selected from the group consisting of a porphyrin compound, a chlorin compound, a bacteriochlorin compound, a phthalocyanine compound, and a cyanine dye.

3. The photosensitive substance-containing lipoprotein according to claim 1, wherein the photosensitive substance is a porphyrin compound.

4. The photosensitive substance-containing lipoprotein according to claim 3, wherein the porphyrin compound has a porphyrin ring and a hydrophilic group.

5. The photosensitive substance-containing lipoprotein according to claim 3, wherein the porphyrin compound is verteporfin.

6. The photosensitive substance-containing lipoprotein according to claim 1, which has a volume average particle diameter of 140 nm or less.

7. The photosensitive substance-containing lipoprotein according to claim 1, wherein the vascular endothelial cell-targeting peptide or aptamer is a neovascular endothelial cell-targeting peptide or aptamer.

8. The photosensitive substance-containing lipoprotein according to claim 7, wherein the neovascular endothelial cell-targeting peptide is one type or two or more types selected from the group consisting of an NGR-containing peptide, an RGD-containing peptide, and a CPRECES-containing peptide.

9. The photosensitive substance-containing lipoprotein according to claim 1, wherein the cell affinity peptide is one type or two or more types selected from the group consisting of a TAT peptide, penetratin (PEN), polyarginine (R8), LL-37, transportan, Pep-1, and MTS.

10. A photodynamic therapy preparation, comprising the photosensitive substance-containing lipoprotein according to any one of claims 1 to 9.

11. The photodynamic therapy preparation according to claim 10, which is used in the treatment of age-related macular degeneration.

12. The photodynamic therapy preparation according to claim 10 or 11, wherein the photodynamic therapy comprises administering the photosensitive substance-containing lipoprotein to a subject who is affected with age-related macular degeneration, and applying light irradiation to a new blood vessel generation site in the posterior eye segment.

13. A method for occluding new blood vessels in the posterior eye segment, wherein the method comprises administering the photosensitive substance-containing lipoprotein according to any one of claims 1 to 9 to a subject who is affected with age-related macular degeneration, and applying light irradiation to a new blood vessel generation site in the posterior eye segment.

14. The photodynamic therapy preparation according to claim 10, which is used in the treatment of malignant tumor.

15. The photodynamic therapy preparation according to claim 14, wherein the photodynamic therapy comprises administering the photosensitive substance-containing lipoprotein to a subject having malignant tumor, and applying light irradiation to a new blood vessel generation site in the malignant tumor part.

16. A method for occluding new blood vessels in a malignant tumor part, wherein the method comprises administering the photosensitive substance-containing lipoprotein according to any one of claims 1 to 9 to a subject having malignant tumor, and applying light irradiation to a new blood vessel generation site in the malignant tumor part.

[Figure 1]

**VP@eLP1**
NGR(−)

**VP@eLP2**
NGR(+)

**Vis**

[Figure 2]

[Figure 3]

[Figure 4]

[Figure 5]

[Figure 6]

[Figure 7]

[Figure 8]

[Figure 9]

[Figure 10]

PBS+PDT

VP@eLP2+PDT

Vis+PDT

[Figure 11]

[Figure 12]

*p < 0.05 by welch's t-test (vs. Bestatin(−))

[Figure 13]

[Figure 14]

## INTERNATIONAL SEARCH REPORT

| International application No. |
| --- |
| **PCT/JP2024/031349** |

**A. CLASSIFICATION OF SUBJECT MATTER**

*C07K 14/775*(2006.01)i; *A61K 38/41*(2006.01)i; *A61K 41/00*(2020.01)i; *A61P 27/02*(2006.01)i; *A61P 43/00*(2006.01)i; *C07K 14/795*(2006.01)i; *C12N 15/12*(2006.01)i

FI: C07K14/775 ZNA; A61K41/00; A61P27/02; A61K38/41; A61P43/00 121; C07K14/795; C12N15/12

According to International Patent Classification (IPC) or to both national classification and IPC

**B. FIELDS SEARCHED**

Minimum documentation searched (classification system followed by classification symbols)

C07K14/775; A61K38/41; A61K41/00; A61P27/02; A61P43/00; C07K14/795; C12N15/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2021-138629 A (UNIV TOYAMA PREFECTURAL) 16 September 2021 (2021-09-16) claims, examples | 1-16 |
| Y | JP 2017-537117 A (BAYER HEALTHCARE LLC) 14 December 2017 (2017-12-14) claims, examples | 1-16 |
| Y | JP 2008-524224 A (LIGHT SCIENCES ONCOLOGY INC.) 10 July 2008 (2008-07-10) claims, examples | 1-16 |

☐ Further documents are listed in the continuation of Box C.　　☑ See patent family annex.

| | |
| --- | --- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **28 October 2024** | **19 November 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/031349**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/JP2024/031349**

| Patent document cited in search report | | | Publication date (day/month/year) | Patent family member(s) | | | Publication date (day/month/year) |
|---|---|---|---|---|---|---|---|
| JP | 2021-138629 | A | 16 September 2021 | (Family: none) | | | |
| JP | 2017-537117 | A | 14 December 2017 | WO claims, examples | 2016/090590 | A1 | |
| JP | 2008-524224 | A | 10 July 2008 | WO claims, examples | 2006/065727 | A1 | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017537117 A
- JP 2008524224 A

- JP 2021138629 A

**Non-patent literature cited in the description**

- Guidelines for Photodynamic Therapy for Age-related Macular Degeneration. *Journal of Japanese Ophthalmological Society*, vol. 108 (4)
- **ANTONIO M. GOTTO, JR. et al.** *Methods Enzymol.*, 1986, vol. 128, 3-41

- **J. LAHDENRANTA et al.** *The FASEB Journal*, 2007, vol. 21, 3272-3278
- *NUCLEIC ACID THERAPEUTICS*, 2015, vol. 25 (5), 227-234
- **GUO et al.** *Oncol. Res.*, 2020, vol. 28, 533-540